# EUROPÄISCHE PATENTSCHRIFT

(11) **EP 0 388 758 B1**
(45) Veröffentlichungstag und Bekanntmachung des Hinweises auf die Patenterteilung: **02.08.2000**
(21) Anmeldenummer: 90104700.1
(22) Anmeldetag: 13.03.1990
(51) Int. Cl.: C12N 15/87, A61K 47/48

(54) **Neue Protein-Polykation-Konjugate**
Protein-polycation conjugate
Conjugé protéine-polycation

(30) Priorität: 16.03.1989 AT 61089
(43) Veröffentlichungstag der Anmeldung: 26.09.1990
(73) Patentinhaber: BOEHRINGER INGELHEIM INTERNATIONAL GmbH, 55218 Ingelheim am Rhein (DE)
(72) Erfinder: Beug, Hartmut, Dr., A-1140 Wien (AT); Birnstiel, Max L., Prof. Dr., A-1100 Wien (AT); Cotten, Matthew, Dr., A-1130 Wien (AT); Wagner, Ernst, Dr., A-2103 Langenzersdorf (AT)

(56) Entgegenhaltungen:
- EP-A- 0 153 151
- WO-A-88/00837
- WO-A-88/05077
- CELLULAR & MOLECULAR BIOLOGY, Band 28, Nr. 1, 1982, Seiten 15-18, Pergamon Press Ltd, GB; J.C. STAVRIDIS et al.: "Use of transferrin as a gene-carrier to the erythroid cells of the marrow"
- THE JOURNAL OF BIOLOGICAL CHEMISTRY, Band 262, Nr. 10, 5. April 1987, Seiten 4429-4432, The American Society of Biological Chemists, Inc., US; G.Y. WU et al.: "Receptor-mediated in vitro gene transformation by a soluble DNA carrier system"
- CHEMICAL ABSTRACTS, Band 106, Nr. 23, 8. Juni 1987, Seite 205, Zusammenfassung 190339v, Columbus, Ohio, US; U. WIENHUES et al.: "A novel method for transfection and expression of reconstituted DNA-protein complexes in eukaryotic cells", & DNA 1987, 6(1), 81-9
- PROC. NATL. ACAD. SCI. USA, Band 87, Mai 1990, Seiten 3410-3414; E. WAGNER et al.: "Transferrin-polycation conjugates as carriers for DNA uptake nto cells"

## Beschreibung

Die Erfindung bezieht sich auf den Transport von zu Polykationen affinen Substanzen, insbesondere Nukleinsäuren, in die Zelle, wobei der Transport über Rezeptor-vermittelte Endozytose abläuft.

Antisense RNAs und DNAs haben sich in zellfreien Systemen sowie innerhalb der lebenden Zelle als wirksame Mittel für die selektive Inhibierung bestimmter Gensequenzen erwiesen. Die Wirkungsweise beruht in der spezifischen Erkennung eines komplementären Nukleinsäurestranges und Anlagerung daran, wodurch Transkription, Translation und Spaltprozesse beeinträchtigt werden. Dieser Wirkmechanismus ermöglicht grundsätzlich die Anwendung von Antisense-Oligonukleotiden als Therapeutika, die die Expression bestimmter Gene (wie deregulierte Onkogene oder virale Gene) in vivo blockieren. Es wurde bereits gezeigt, daß kurze Antisense- Oligonukleotide in Zellen importiert werden und dort ihre inhibierende Wirkung ausüben können (Zamecnik et al., 1986), wenngleich ihre intrazelluläre Konzentration, u.a. wegen ihrer beschränkten Aufnahme durch die Zellmembran auf Grund der starken negativen Ladung der Nukleinsäuren, gering ist.

Ein weiterer Ansatz zur selektiven Inhibierung von Genen besteht in der Anwendung von Ribozymen, das sind RNA-Moleküle, die bestimmte RNA-Sequenzen erkennen, daran binden und spalten können. Auch hier besteht das Bedürfnis, eine möglichst hohe Konzentration von aktiven Ribozymen in der Zelle zu gewährleisten, wofür der Transport in die Zelle einer der limitierenden Faktoren ist.

Um diesem limitierenden Faktor zu begegnen, wurden bereits mehrere Lösungen vorgeschlagen.

Eine dieser Lösungswege besteht in direkten Modifikationen der Nukleinsäuren, z.B. durch Substitution der geladenen Phosphodiestergruppen durch ungeladene Methylphosphonate (Smith et al., 1986), Carbamate (Stirchak et al., 1987) oder Silylverbindungen (Cormier et al., 1988) oder durch Phosphorothioate (Stern et al., 1988). Eine weitere Möglichkeit der direkten Modifikation besteht in der Verwendung von Nukleosidanalogen (Morvan et al., 1988, Praseuth et al., 1988)).

Wenn auch einige dieser Vorschläge einen grundsätzlich vielversprechenden Ansatz der Lösung des Problems darstellen, weisen sie doch verschiedene Nachteile auf, z.B. eine geringere Bindung an das Zielmolekül sowie einen geringeren Grad der eigentlichen Hemmwirkung. Ein prinzipieller Nachteil bei der in vivo Anwendung von modifizierten Oligonukleotiden besteht weiters in der möglichen Toxizität dieser Verbindungen.

Ein alternativer Ansatz zur direkten Modifikation der Oligonukleotide besteht darin, das Oligonukleotid als solches unverändert zu belassen und mit einer Gruppe zu versehen, die ihm die angestrebten Eigenschaften verleiht, z.B. mit Molekülen, die den Transport in die Zelle erleichtern. Einer der Lösungsvorschläge im Rahmen dieses Prinzips besteht darin, das Oligonukleotid mit polykationischen Verbindungen zu konjugieren (Lemaitre et al., 1987).

Für die Gentransformation von Säugetierzellen in vitro sind verschiedene Techniken bekannt, deren Anwendbarkeit in vivo jedoch beschränkt ist (dazu zählen das Einbringen von DNA mittels Viren, Liposomen, Elektroporation, Mikroinjektion, Zellfusion, DEAE-Dextran oder die Calciumphosphat-Präzipitationsmethode).

Es wurde daher bereits versucht, ein in vivo anwendbares lösliches System zu entwickeln, das DNA zielgerichtet über Rezeptor-vermittelte Endozytose in die Zellen befördert (G.Y. Wu, C.H. Wu, 1987). Dieses System wurde für Hepatozyten entwickelt und beruht im wesentlichen auf den beiden folgenden Gegebenheiten:
1. An der Hepatozytenoberfläche existieren Rezeptoren, die bestimmte Glykoproteine binden und in die Zelle befördern.
2. DNA kann durch starke elektrostatische Wechselwirkung unter Ausbildung von löslichen Komplexen an polykationische Verbindungen, z.B. Polylysin, gebunden werden.

Das System beruht auf dem Prinzip, Polylysin an ein solches Glykoprotein, auf das der Rezeptor anspricht, zu koppeln und daraufhin durch Zugabe von DNA einen löslichen Glykoprotein/Polylysin/DNA-Komplex zu bilden, der zu den den Glykoprotein-Rezeptor aufweisenden Zellen transportiert wird und nach Aufnahme des Komplexes in die Zelle die Expression der DNA-Sequenz ermöglicht.

Stavridis und Psallidopoulos, 1982, schlugen, Transferrin als Trägermolekül für den Transport von genetischem Material vor, wobei Konjugate verwendet wurden, die durch Kopplung eines Histongemisches an Transferrin erhalten wurden.

Transferrine sind eine Klasse verwandter metallbindender Transportglykoproteine mit in vivo Spezifität für Eisen. Es wurden verschiedene Säugetiertransferrine identifiziert, wobei das Plasma-Transferrin die meisten Körpergewebe mit Eisen versorgt. Hauptproduzent für Transferrin ist die Leber.

Transferrin weist ein Molekulargewicht von ca. 80000 auf, wovon 6% auf die Zuckerreste entfallen. Ein Transferrinmolekül kann zwei Moleküle Eisen binden, wobei die Bindung die Anwesenheit von Karbonat- oder Bikarbonationen erfordert.

Der Transferrin-Rezeptor, von dem es möglicherweise auf den ver-schiedenen Zellen geringfügig - etwa in der Kohlenhydratgruppe - voneinander abweichende Formen gibt, ist ein Transmembran-glykoprotein mit einem Molekulargewicht von ca. 180000, wobei ein Rezeptormolekül ein oder möglicherweise zwei Moleküle Transferrin binden kann.

Der Transferrin-Rezeptor hat beim physiologischen pH-Wert von 7,4 eine sehr hohe Affinität für das Fe₂-Transferrin, geringere Affinität für das Fe-Transferrin und praktisch keine Affinität für das Apotransferrin, wenngleich dieses bei pH ≈5 mit dem Rezeptor einen sehr stabilen Komplex bildet.

Transferrin-Rezeptoren wurden besonders zahlreich in Vorläufern von Erythrozyten, Plazenta und Leber, in meßbarer Anzahl auch in vielen anderen Körpergeweben nachgewiesen. Von besonderem Interesse ist die Beobachtung, daß der Rezeptor in wachsenden Zellen hochreguliert ist. Es wurde auch beobachtet, daß die Rezeptorzahl in neoplastischem Gewebe in vivo gegenüber benignen Läsionen wesentlich erhöht ist, was auf einen erhöhten Eisenbedarf hinweist. Der Aufnahmemechanismus des Transferrin-Eisen-Komplexes durch Rezeptoren und sein intrazellulärer Zyklus sind gut untersucht.

Über den exakten Ablauf der Abgabe der Eisenmoleküle durch Transferrin besteht noch keine vollständige Klarheit, wenngleich die Ansicht überwiegt, daß die Abgabe vorwiegend intrazellulär erfolgt. In einem energie- und temperaturabhängigen Vorgang werden Fe-Transferrin bzw. Fe₂-Transferrin an der Zellmembran an den Rezeptor gebunden. Daraufhin wird der Komplex in ein Vesikel, genannt Endosom oder Rezeptosom, aufgenommen. Dieses wird mit einem weiteren Vesikel, das einen pH-Wert von < 5,5 aufweist, vereinigt; die resultierende Ansäuerung bewirkt die Freigabe des Eisens vom Transferrin. Der Apotransferrin-Rezeptor-Komplex wird daraufhin an die Zellmembran zurücktranportiert, wo der neutrale pH-Wert die Loslösung des Apotransferrins vom Rezeptor ins umgebende Medium bewirkt. Es gibt Hinweise dafür, daß das Recycling, dessen Funktionieren auf der bei saurem und neutralem pH-Wert unterschiedlichen Affinität des Rezeptors für Apo- bzw. Eisentransferrin beruht, über Vesikel des Golgi-Apparates erfolgt.

Auf molekularer Ebene ist die Auslösung des Transferrin-Zyklus noch nicht geklärt; lediglich hinsichtlich einiger Aspekte gibt es Hinweise, z.B. zur möglichen Rolle der Phosphorylierung (Huebers et al., 1987).

Aufgabe der vorliegenden Erfindung war es, neue definierte Transferrin-Polykation-Konjugate bereitzustellen.

Diese Aufgabe wurde gelöst durch Transferrin-Konjugate, in welchen Histone durch andere Polykationen ersetzt sind.

Die vorliegende Erfindung betrifft ein Konjugat, bestehend aus Transferrin und einem daran kovalent gekoppelten Polykation, das dadurch gekennzeichnet ist, daß das Polykation ein synthetisches homologes Polypeptid oder ein Polyethylenimin ist.

Es wurde überraschend festgestellt, daß mit Hilfe der erfindungsgemäßen Konjugate Nukleinsäuren unter Beibehaltung ihrer inhibierenden Aktivität effizient in die Zelle transportiert werden können.

Unter "Transferrin" sind im Rahmen der vorliegenden Erfindung sowohl die natürlichen Transferrine zu verstehen als auch Transferrin-Modifikationen, die vom Rezeptor gebunden und in die Zelle transportiert werden (solche Modifikationen können beispielsweise in einer Änderung von Aminosäuren oder in einer Verkürzung des Moleküls auf den für die Rezeptorbindung maßgeblichen Anteil bestehen).

Das molare Verhältnis Transferrin:Polykation beträgt bevorzugt 10:1 bis 1:4, wobei zu berücksichtigen ist, daß es zur Ausbildung von Aggregaten kommen kann. Dieses Verhältnis kann jedoch bei Bedarf innerhalb weiterer Grenzen liegen, solange die Bedingung erfüllt ist, daß Komplexierung der Nukleinsäure(n) stattfindet und gewährleistet ist, daß der gebildete Komplex durch den Transferrinrezeptor gebunden und in die Zelle befördert wird, was durch einfach durchzuführende Versuche von Fall zu Fall überprüft werden kann.

Das jeweils gewählte Verhältnis richtet sich vor allem nach der Größe des Polykationmoleküls sowie nach der Anzahl und Verteilung der positiv geladenen Gruppierungen, Kriterien, die auf Größe, Struktur sowie allenfalls vorhandene Modifikationen der zu transportierenden Nukleinsäure(n) abgestimmt werden. Die Polykationen können gleich oder verschieden sein.

Als Polykationen können folgende Verbindungen verwendet werden:
a) Synthetische Polypeptide, wie homologe Polypeptide (Polylysin, Polyarginin) oder heterologe Polypeptide (bestehend aus zwei oder mehr Vertretern positiv geladener Aminosäuren).
b) Polyethylenimine.
   Die Größe der Polykationen wird bevorzugt so gewählt, daß die Summe der positiven Ladungen etwa 20 bis 500 beträgt, sie wird auf die zu transportierende Nukleinsäure abgestimmt.

Die erfindungsgemäßen Transferrin-Polykation-Konjugate können auf chemischem oder, falls das Polykation ein Polypeptid ist, auf rekombinantem Weg hergestellt werden. Die Kopplung auf chemischem Weg kann in für die Kopplung von Peptiden an sich bekannter Weise erfolgen, wobei, falls erforderlich, die Einzelkomponenten vor der Kopplungsreaktion mit Linkersubstanzen versehen werden (diese Maßnahme ist dann erforderlich, wenn von vornherein keine für die Kopplung geeignete funktionelle Gruppe, z.B. eine Mercapto- oder Alkoholgruppe, verfügbar ist. Bei den Linkersubstanzen handelt es sich um bifunktionelle Verbindungen, die zunächst mit funktionellen Gruppen der Einzelkomponenten zur Reaktion gebracht werden, worauf die Kopplung der modifizierten Einzelkomponenten durchgeführt wird.)

Je nach gewünschten Eigenschaften der Konjugate, insbesondere im Hinblick auf deren Stabilität, kann die Kopplung erfolgen über
a) Disulfidbrücken, die unter reduzierenden Bedingungen wieder gespalten werden können (z.B. bei Verwendung von Succinimidylpyridyldithiopropionat (Jung et al., 1981).
b) Unter biologischen Bedingungen weitgehend stabile Verbindungen (z.B. Thioether durch Reaktion von Maleimido-Linkern mit Sulfhydrylgruppen des an die zweite Komponente gebundenen Linkers).
c) Unter biologischen Bedingungen labile Brücken, z.B. Esterbindungen, oder unter schwach sauren Bedingungen instabile Acetal- oder Ketalbindungen.

Die Herstellung der erfindungsgemäßen Konjugate auf rekombinantem Weg bietet den Vorteil, genau definierte, einheitliche Verbindungen erhalten zu können, während bei der chemischen Kopplung Konjugat-Gemische entstehen, die aufgetrennt werden müssen.

Die rekombinante Herstellung der erfindungsgemäßen Konjugate ist nach für die Herstellung von chimären Polypeptiden bekannten Methoden durchführbar. Dabei können die polykationischen Peptide hinsichtlich ihrer Größe und Aminosäuresequenz variiert werden. Die gentechnologische Herstellung bietet auch den Vorteil, den Transferrin-Anteil des Konjugats modifizieren zu können, indem z.B. durch geeignete Mutationen die Bindungsfähigkeit an den Rezeptor gesteigert werden kann oder indem der Transferrinanteil auf den für die Bindung an den Rezeptor maßgeblichen Teil des Moleküls verkürzt wird. Besonders zweckmäßig für die rekombinante Herstellung der erfindungsgemäßen Konjugate ist die Verwendung eines Vektors, der die für den Transferrinanteil kodierende Sequenz sowie einen Polylinker enthält, in den die jeweils benötigte für das polykationische Peptid kodierende Sequenz eingesetzt wird. Auf diese Weise kann ein Satz von Expressionsplasmiden erhalten werden, von dem bei Bedarf das die gewünschte Sequenz enthaltende zur Expression des erfindungsgemäßen Konjugats herangezogen wird.

Bei den in die Zelle zu transportierenden Nukleinsäuren kann es sich um DNAs oder RNAs handeln, wobei hinsichtlich der Nukleotidsequenz keine Beschränkungen bestehen. Die Nukleinsäuren können modifiziert sein, sofern die Modifikation den polyanionischen Charakter der Nukleinsäuren nicht beeinträchtigt; zu diesen Modifikationen zählen z.B. die Substitution der Phosphodiestergruppe durch Phosphorothioate oder in der Verwendung von Nukleosidanalogen.

Als Nukleinsäuren kommen im Rahmen der vorliegenden Erfindung vor allem diejenigen in Betracht, die zum Zweck der Inhibierung spezifischer Gensequenzen in die Zelle transportiert werden sollen. Dazu zählen Antisense-Oligonukleotide und Ribozyme, gegebenenfalls zusammen mit einer Carrier-Nukleinsäure.

Bezüglich der Größe der Nukleinsäuren ermöglicht die Erfindung ebenfalls Anwendungen in einem weiten Bereich. Hinsichtlich der unteren Grenze besteht keine durch das erfindungsgemäße Transportsystem bedingte Limitierung; eine etwaige Begrenzung nach unten ergibt sich aus anwendungsspezifischen Gründen, weil z.B. Antisense-Oligonukleotide unter etwa 10 Nukleotiden auf Grund zu geringer Spezifität kaum für die Anwendung in Frage kommen. Mit Hilfe der erfindungsgemäßen Konjugate können auch Plasmide in die Zelle befördert werden, wobei vor allem kleinere Plasmide, die in ihrer Funktion als Carrier-Nukleinsäure (etwa retrovirale Vektoren mit bis zu 5000 bp) zur Anwendung kommen, von praktischer Bedeutung sind.
Es ist auch möglich, gleichzeitig verschiedene Nukleinsäuren mit Hilfe der erfindungsgemäßen Konjugate in die Zelle zu befördern.
Ein weiterer Vorteil der vorliegenden Erfindung liegt in dem Umstand, daß für Transferrin und den Rezeptor Polymorphismen bestehen, die für den zielgerichteten Transport inhibierender Nukleinsäuren zu bestimmten Zellen ausgenutzt werden können.
Im Rahmen der vorliegenden Erfindung konnte gezeigt werden, daß die Transferrin-Polykation-Konjugate effizient in lebende Zellen aufgenommen und internalisiert werden. Die erfindungsgemäßen Konjugate bzw. Komplexe sind für das Zellwachstum nicht schädlich. Dies ermöglicht eine wiederholte Verabreichung und damit einen konstant hohe Expression in die Zelle eingeführter Gene.
Weiters konnte nachgewiesen werden, daß die Polykation-Transferrin-Konjugate den nativen Transferrin-Eisenkomplex funktionell ersetzen können.
Daß die Transferrin-Polykation/DNA-Komplexe durch die Zelle über den Transferrin-Rezeptor aufgenommen werden, wurde mit Hilfe des Luciferase-Gens als DNA-Komponente bestätigt. Es wurde gezeigt, daß natives Transferrin effizient den Transferrin-Polykation/DNA-Komplex verdrängt, was anhand der Verringerung der Luciferaseaktivität in der Zelle gemessen wurde.

Durch die im Rahmen der vorliegenden Erfindung durchgeführten Versuche konnte auch gezeigt werden, daß ein tRNA-Ribozymgen (das Ribozym war gegen eine V-erbB Sequenz gerichtet) mit Hilfe eines erfindungsgemäßen Konjugats (Polylysin-Transferrin) in erbB-transformierte Hühnerzellen eingebracht und die transformierende Wirkung des Onkogens schwächen kann. Dieses Ergebnis ist umso bedeutsamer, als in diesen Versuchen nur eine geringe Menge an Ribozymgen verwendet wurde.

Das Verhältnis Nukleinsäure: Konjugat kann innerhalb eines weiten Bereichs schwanken, wobei es nicht unbedingt erforderlich sein muß, sämtliche Ladungen der Nukleinsäure zu neutralisieren. Dieses Verhältnis wird von Fall zu Fall nach Kriterien wie Größe und Struktur der zu transportierenden Nukleinsäure, Größe des Polykations, Anzahl und Verteilung seiner Ladungen, derart einzustellen sein, daß ein für die jeweilige Anwendung günstiges Verhältnis zwischen Transportfähigkeit und biologischer Aktivität der Nukleinsäure besteht. Dieses Verhältnis kann zunächst grob eingestellt werden, etwa an Hand der Verzögerung der Wanderungsgeschwindigkeit der DNA in einem Gel (z.B. mittels "Mobility Shift" auf einem Agarosegel) oder durch Dichtegradientenzentrifugation. Nach Erhalt dieses vorläufigen Verhältnisses kann es zweckmäßig sein, im Hinblick auf die in der Zelle maximal verfügbare Aktivität der Nukleinsäure Transportversuche mit dem radioaktiv markierten Komplex durchzuführen und den Konjugat-Anteil gegebenenfalls derart zu reduzieren, daß die verbleibenden negativen Ladungen der Nukleinsäure dem Transport in die Zelle nicht hinderlich sind.

Die Herstellung der Transferrin-Polykation/Nukleinsäure-Komplexe, die ebenfalls Gegenstand der Erfindung sind, kann nach in an sich für die Komplexierung polyionischer Verbindungen bekannten Methoden vorgenommen wurden. Eine Möglichkeit, unkontrollierte Aggregation bzw. Ausfällung zu vermeiden, besteht darin, die beiden Komponenten zunächst bei hoher (etwa 1 molarer) Kochsalzkonzentration zu mischen, und anschließend durch Dialyse oder Verdünnung auf physiologische Kochsalzkonzentration einzustellen. Bei der Komplexbildungsreaktion werden bevorzugt keine zu hohen DNA- und Konjugatkonzentrationen (mehr als 100 µg/ml) verwendet, um eine Ausfällung der Komplexe zu vermeiden.

Bevorzugt als Nukleinsäureanteil der erfindungsgemäßen Transferrin-Polykation-Nukleinsäurekomplexes ist Antisense-DNA, Antisense-RNA oder ein Ribozym bzw. das dafür kodierende Gen. Bei der Anwendung von Ribozymen und Antisense-RNAs ist es besonders vorteilhaft, die für diese die Funktion von RNA inhibierenden RNAs kodierenden Gene, bevorzugt zusammen mit einem Carriergen, einzusetzen. Durch die Einführung des Gens in die Zelle wird gegenüber dem Import der RNA als solcher eine beträchtliche Amplifikation der RNA und damit ein für die angestrebte Hemmung der biologischen Reaktion ausreichender Vorrat gewährleistet. Besonders geeignete Carriergene sind die für die Transkription durch Polymerase III erforderlichen Transkriptionseinheiten, z.B. tRNA-Gene. In diese können z.B. Ribozymgene derart eingefügt werden, daß bei Transkription das Ribozym Teil eines kompakten Polymerase III-Transkripts ist. Mit Hilfe des Transportsystems gemäß der vorliegenden Erfindung kann die Wirkung dieser genetischen Einheiten verstärkt werden, indem eine erhöhte Ausgangskonzentration des Gens in der Zelle gewährleistet wird.

Gegenstand der Erfindung ist weiters ein Verfahren zum Einführen von Nukleinsäure(n) in menschliche oder tierische Zellen, wobei bevorzugt ein unter physiologischen Bedingungen löslicher Komplex gebildet wird.

Gegenstand der Erfindung sind weiters pharmazeutische Zubereitungen, enthaltend als wirksame Komponente eine ein Gen spezifisch inhibierende Nukleinsäure, komplexiert mit einem Transferrin-Polykation-Konjugat, z.B. in Form eines Lyophilisats. Solche pharmazeutischen Zubereitungen können verwendet werden, um mit Antisense-Oligonukleotiden, Antisense-Oligonukleotidanaloga oder Ribozymen bzw. den dafür kodierenden DNAs, gegebenenfalls zusammen mit einer Carriernukleinsäure, im menschlichen oder tierischen Organismus pathogene Viren, wie HIV oder verwandte Retroviren, Oncogene oder andere Schlüsselgene, die das Wachstum und/oder die Differenzierung von Zellen kontrollieren, z.B. das c-fos Gen oder das c-myc Gen, zu inhibieren.

Ein weiteres Anwendungsgebiet liegt in der Bekämpfung von Krankheiten durch Hemmung der Produktion von unerwünschten Genprodukten, z.B. des bei der Alzheimer-Krankheit auftretenden Haupt-Plaqueproteins ("major plaque protein") oder Autoimmunerkrankungen verursachender Proteine.

Die Erfindung wird anhand der nachfolgenden Beispiele illustriert.

### Beispiel 1

### Herstellung von Transferrin - Polylysin 90 - Konjugaten

Die Kopplung erfolgte analog literaturbekannter Methoden (Jung et al., (1981)) durch Einführung von Disulfidbrücken nach Modifizierung mit Succinimidylpyridyldithiopropionat.

### a) 3-(2-Pyridyldithio)propionat - modifiziertes Transferrin:

6 ml einer über Sephadex G-25 gelfiltrierten Lösung von 120 mg ( 1.5 µMol) Transferrin ( aus Hühnereiweiß, Sigma, Conalbumin Type I, eisenfrei) in 3 mI 0.1 M Natriumphosphat-Puffer (pH 7.8) wurden unter starkem Schütteln mit 200 µl einer 15 mM ethanolischen Lösung von Succinimidyl 3-(2-pyridyldithio)propionat (3 µM, SPDP, Pharmacia) versetzt und 1 h bei Raumtemperatur und gelegentlichem Schütteln reagieren gelassen. Über eine Gelsäule (Sephadex G-25, 14 x 180 mm, 0.1 M Natriumphosphat-Puffer pH 7.8 ) wurden niedermolekulare Reaktionsprodukte und Reagensreste abgetrennt und dabei 7 ml der Produktfraktion erhalten; der Gehalt von an Transferrin gebundenem Pyridyldithiopropionatresten wurde anhand eines Aliquots nach Reduktion mit Dithiothreitol durch photometrische Bestimmung der Menge an freigesetztem Pyridin-2-thion ermittelt und betrug ca. 2.6 µMol.
In identischer Weise wurde humanes Transferrin (Sigma, eisenfrei) modifiziert.

### b) Mercaptopropionat - modifiziertes Polylysin 90 (pL 90):

Eine Lösung von 18 mg (ca. 1.0 µMol) Poly(L)Lysin - Hydrobromid (Sigma, Fluoresceinisothiocyanat (= FITC) - markiert, Molekulargewicht ca. 18000 / entspricht einem durchschnittlichem Polymerisationsgrad ca. 90) in 3 ml 0.1 M Natriumphosphat (pH 7.8) wurde über Sephadex G-25 filtriert (die Fluoreszensmarkierung wurde in Natriumbicarbonatpuffer pH 9 während 3h durchgeführt). Die Polylysin-Lösung wurde mit Wasser auf 7 ml verdünnt, unter gutem Schütteln mit 270 µl einer 15 mM ethanolischen Lösung von SPDP versetzt, und 1 h in der Dunkelheit bei Raumtemperatur und unter gelegentlichem Schütteln reagieren gelassen. Nach Zugabe von 0.5 ml 1 M Natriumacetat-Puffer (pH 5.0) wurde zur Abtrennung von niedermolekularen Substanzen über Sephadex G-25 filtriert (Eluens : 20 mM Natriumacetat-Puffer pH 5.0). Die Produktfraktion (Ninhydrinfärbung, Fluoreszenz) wurde im Vakuum eingeengt, mit Puffer auf pH ca. 7 gebracht, eine Lösung von 23 mg (150 µMol) Dithiothreitol in 200 µl Wasser zugegeben und 1 h bei Raumtemperatur unter Argon in der Dunkelheit stehen gelassen. Durch weitere Gelfiltration (Sephadex G-25, 14 x 130 mm Säule, 10 mM Natrium-acetat-Puffer pH 5.0) wurde von überschüssigem Reduktionsmittel abgetrennt und man erhielt 3.5 ml Produktlösung an Fluoreszenzmarkiertem Polylysin mit einem Gehalt von 3.8 µMol Mercaptogruppen (photometrische Bestimmung mittels Ellman's Reagens, 5,5′-Dithiobis(2-nitrobenzoesäure).

### c) Transferrin - Polylysin - Konjugate:

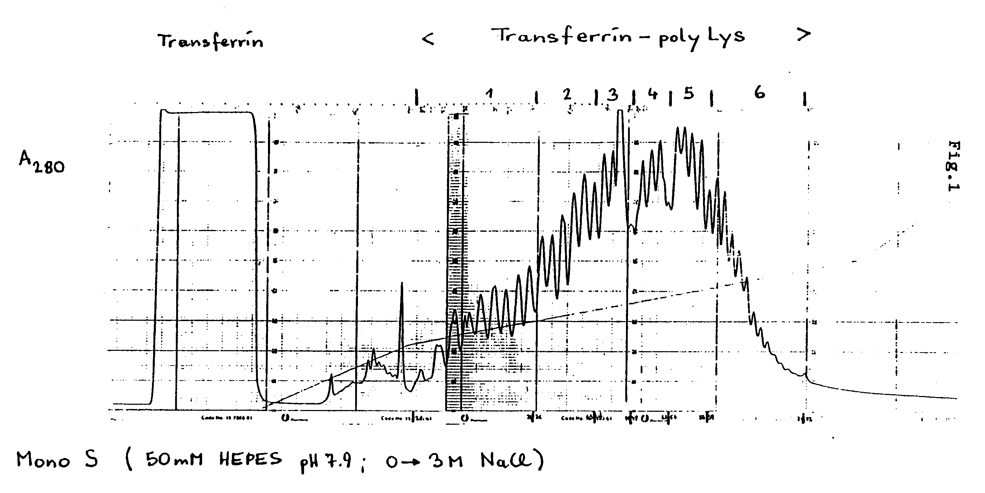

Die unter a) erhaltene Lösung von modifiziertem Transferrin ( 7 ml in 0.1 M Natriumphosphat-Puffer pH 7.8, ca. 1.5 µMol Transferrin mit ca. 2.6 µMol Pyridyldithiopropionat-Resten) wurde mit Argon gespült; es wurden 2.0 ml der nach b) erhaltenen Lösung von Mercapto-modifiziertem Polylysin (in 10 mM Natriumacetat-Puffer pH 5.0, das entspricht ca. 0.6 µMol Polylysin mit ca. 2.2 µMol Mercaptogruppen) zugegeben, mit Argon gespült, geschüttelt und 18 h bei Raumtemperatur in der Dunkelheit und unter Argon reagieren gelassen. Das Reaktionsgemisch wurde mit Wasser auf 14 ml verdünnt und durch Ionenaustauschchromatographie aufgetrennt (Pharmacia Mono S Säule HR 10/10, Gradienteneluation, Puffer A : 50 mM HEPES pH 7.9 , Puffer B : A + 3 M Natriumchlorid, 0.5 ml/min, Fig. 1). Nichtkonjugiertes Transferrin wurde am Anfang eluiert, Produktfraktionen bei ca. 0.66 - 1.5 M Natriumchlorid.

Es wurden, über sämtliche Fraktionen gemittelt, Konjugate erhalten, die ein Verhältnis Transferrin-Polylysin von 1,3:1 aufwiesen.

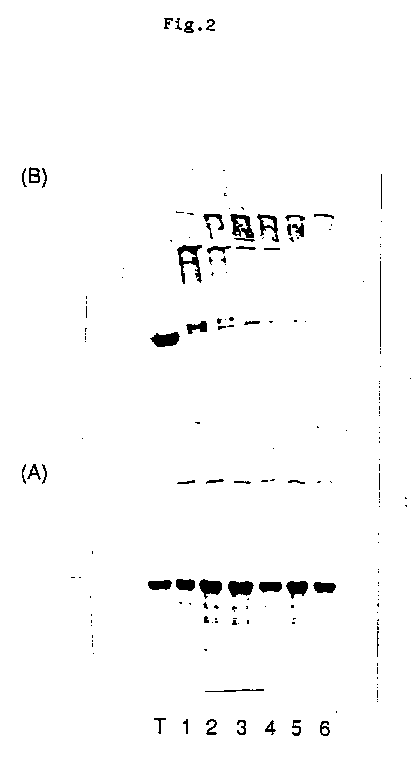

Die konjugierten Produkte (Ninhydrinfärbung, in UV bei 280 nm Proteinabsorbtion, und Fluoreszenzmessung FITC-markierten Polylysins bei 495 nm) wurden in 6 Fraktionen mit einem Gehalt von je ca. 10 mg Transferrin gesammelt. Die Fraktionen wurden zunächst gegen eine 100 mM Eisen(III)citrat-Lösung ( mit Natriumhydrogencarbonat auf pH 7.8 gebracht) dialysiert und danach noch zweimal gegen 1 mM HEPES-Puffer (pH 7.5).
Natriumdodecylsulfat-Gelelektrophorese ( 10% SDS, 8% Polyacrylamid), s. Fig. 2, zeigte bei Vorbehandlung mit 2-Mercaptoethanol in allen 6 Fraktionen einen ungefähr gleichen Gehalt an Transferrin (Fig. 2A), während in nicht reduzierten Proben keine Banden für freies Transferrin, sondern nur weniger weit wandernde Konjugate sichtbar waren (Fig. 2B, T= unbehandeltes Transferrin; 1-6= Konjugate Fraktionen 1-6).

### Beispiel 2

### Herstellung von Transferrin-Polylysin 270- und Transferrin-Polylysin 450-Konjugaten (pL270 und pL450)

### a) Die Herstellung von modifiziertem Transferrin erfolgte analog Beispiel 1 a)

### b) Herstellung von modifiziertem Polylysin 270 und Polylysin 450

Eine gelfiltrierte Lösung von 0,33 µMol Polylysin 270 (mit einem durchschnittlichen Polymerisationsgrad von 270 Lysinresten, mit oder ohne Fluoreszenzmarkierung; entsprechend 19 mg Hydrobromid-Salz) in 1,2 ml 75 mM Natriumacetatpuffer wurde durch Zusatz von Natriumcarbonatpuffer auf pH 8,5 eingestellt. 182 µl einer 15 mM ethanolischen Lösung von SPDP (1,9 µMol wurden unter starkem Rühren hinzugefügt. Eine Stunde später wurden 200 µl 1 M Natriumacetat pH 5 zugefügt; nach Gelfiltration mit 20 mM Natriumacetat wurde eine Lösung erhalten, die 0,27 µMol Polylysin 270 mit 1,3µMol Mercaptogruppen (4,8 Linker pro Polylysinkette) enthielt.
In analoger Weise wurden 0,20 µMol Polylysin 450 (mit einem durchschnittlichen Polymerisationsgrad von 450 Lysinresten) mit 2,25 µMol SPDP modifiziert, wobei ein Produkt von 0,19 µMol Polylysin 450 mit 2,1 µMol Mercaptogruppen (11 Linker pro Polylysinkette) erhalten wurde. Analog Beispiel 1 b) wurden die Dithiopyridingruppen mit Dithiothreitol reduziert, um die freien Sulfhydrylkomponenten zu erhalten.

### c) Herstellung von Transferrin-Polylysin-Konjugaten

Transferrin-Polylysin 270-Konjugate wurden hergestellt, indem 1,0 µMol modifiziertes Transferrin in 100 mM Phosphatpuffer pH 7,8 mit 0,14 µMol modifizierten Polylysins 270 (in 20 mM Natriumacetatpuffer) unter Sauerstoffausschluß in einer Argonatmosphäre gemischt wurden. Nach 18 h bei Raumtemperatur wurde die Reaktionsmischung mit Wasser auf ein Volumen von 10 ml verdünnt und durch Kationenaustausch-Chromatographie (Pharmacia Mono S Säule HR 10/10; Gradientenelution, Puffer A : 50 mM HEPES pH 7,9 ; Puffer B : A + 3 M Natriumchlorid; UV-Absorption bei 280 nm und Fluoreszenzmessung, Anregung 480 nm, Emission 530 nm) fraktioniert. Dabei wurde der Überschuß von nicht gekoppeltem Transferrin zuerst eluiert; die Produktfraktionen wurden zwischen 30 % und 50 % Gradient B eluiert und in 3 Konjugatfraktionen gepoolt (molare Verhältnisse Transferrin zu Polylysin: Pool A: 5,5 zu 1 ; Pool B: 3,4 zu 1 ; Pool C: 1,8 zu 1). Die Konjugate wurden in einer durchschnittlichen Ausbeute von 0,23 µMol Transferrin mit 0,075 µMol Polylysin 270 erhalten.

In ähnlicher Weise wurden Transferrin-Polylysin 450 Konjugate hergestellt, wobei von 1,2 µMol modifiziertem Transferrin gemäß Beispiel 1 a) (in 20 mM HEPES pH 7,9 enthaltend 80 mM Natriumchlorid) und 71 nMol Mercaptomodifiziertem Polylysin 450 gemäß Beispiel 2 b) in Acetatpuffer ausgegangen wurde.

Die Reinigung der Reaktionsmischung wurde über Gelpermeationschromatographie (Pharmacia Superose 12 Säule, 1 M Guanidinchlorid pH 7,3) durchgeführt und ergab nach Dialyse (20 mM HEPES pH 7,3, enthaltend 100 mM Natriumchlorid) Transferrin-Polylysin-Konjugate, enthaltend 0,40 µMol Transferrin mit 38 nMol Polylysin 450.

Der Einbau von Eisen wurde durchgeführt, indem den Proben 6 - 12 µl 100 mM Eisencitratpuffer (enthaltend Natriumbicarbonat, eingestellt auf pH 7,8) pro mg Transferrinanteil zugefügt wurden.

### Beispiel 3

### a) Herstellung von Transferrin-Protamin-Konjugaten

Die Herstellung von modifiziertem Tranferrin wurde analog Beispiel 1 a) durchgeführt.

### b) Herstellung von 3-Mercaptopropionat-modifiziertem Protamin

Einer Lösung von 20 mg (3 µMol) Protamintrifluoracetat-Salz (hergestellt durch Ionenaustausch-Chromatographie aus Lachs-Protamin (= Salmin) -Sulfat, Sigma) in 2 ml DMSO und 0,4 ml Isopropanol, enthaltend 2,6 µl (15 µMol) Ethyldiisopropylamin, wurde eine Lösung von 30 µMol SPDP in 250 µl Isopropanol und 250 µl DMSO in mehreren Portionen während einer Stunde zugefügt. Nach 3,5 h bei Raumtemperatur wurde die Lösung im Hochvakuum eingedampft und in 0,5 %iger Essigsäure mit einem Gehalt von 10 % Methanol aufgenommen. Gelfiltration (Sephadex G10; 0,5 %ige Essigsäure mit 10 % Methanol) ergab nach der Lyophilisierung 16 mg (2,5 µMol) Protaminacetat-Salz, modifiziert mit 2,5 µMol Dithiopyridin-Linker. Reduktion von 1,75 µMol Protamin (enthaltend 1,75 µMol Linker) mit 16 mg Dithiothreitol in Natriumbicarbonatpuffer pH 7,5 während 1 h unter Argon, anschließende pH-Einstellung auf 5,2 und Gelfiltration über Sephadex G10 mit 20 mM Natriumacetatpuffer pH 5,2 ergaben eine Protaminlösung, modifiziert mit 1,6 µMol Mercaptopropionat-Linker.

### c) Herstellung von Transferrin-Protamin-Konjugaten

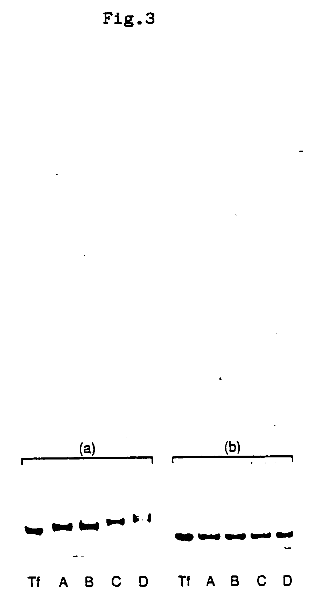

Die Reaktion der nach b) erhaltenen Protaminlösung (1,6 µMol Linker) mit 1,34 µMol Transferrin (modifiziert mit 3,1 µMol Dithiopyridin-Linker) und anschließende Reinigung durch Kationenaustausch-Chromatographie, wie für die Transferrin-Polylysin Konjugate beschrieben, ergaben vier aufeinanderfolgend eluierende Produktfraktionen A - D, enthaltend 90, 320, 240 bzw. 120 nMol modifiziertes Transferrin mit zunehmenden Mengen an Protamin (bestimmt mittels SDS-Gelelektrophorese; 10 % SDS, 8 % Polyacrylamid, Coomassieblau-Färbung. Fig. 3 zeigt das Ergebnis der SDS-Gelelektrophorese. Die Tfprot-Konjugatfraktionen A - D zeigten langsamer wandernde Banden (a), während in β-Mercaptoethanol-reduzierten Proben (b) nur die Transferrinbande sichtbar war. Dialyse und Eiseneinbau wurden, wie für die Transferrin-Polylysin-Konjugate TfpL270 und TfpL450 in Beispiel 2 beschrieben, durchgeführt.

### Beispiel 4

### Herstellung von Komplexen von Transferrin-Polykation-Konjugaten mit DNA

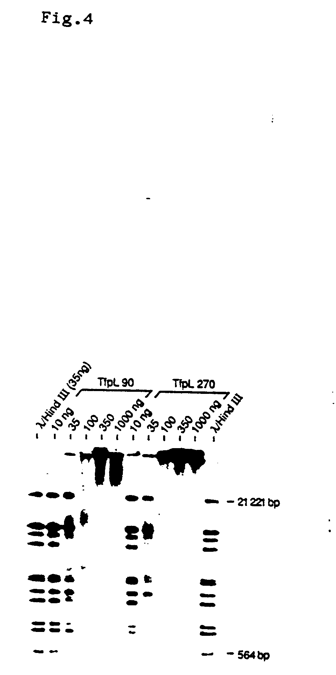

Die Komplexe wurden hergestellt, indem verdünnte Lösungen von DNA (30 µg/ml oder weniger) mit den Transferrin-Polykation-Konjugaten vermischt wurden. Um ein Ausfällen der DNA-Komplexe zu verhindern, wurde phosphatfreier Puffer verwendet (Phosphate verringern die Löslichkeit der Konjugate). Die Bindung der DNA an die Polykation-Konjugate bei physiologischen ionischen Bedingungen wurde durch einen Gel-Mobility-Shift-Assay unter Verwendung von am 3′-Ende ³²P-markierter Lambda DNA, geschnitten mit EcoR I/Hind III, bestätigt (Fig. 4). Zu jeder Probe mit 1 µl (35 ng) DNA wurden 3 µl eines 100 mM HEPES Puffer pH 7,9, enthaltend 1 M Natriumchlorid, gefügt und die Proben mit steigenden Mengen (10 ng bis 1000 ng) von Transferrin-Konjugaten in 11 µl wässeriger Lösung vermischt, was eine Endkonzentration an Natriumchlorid von 200 mM ergab. Elektrophorese auf einem 1 %igen Agarose-Gel mit 1 x TAE Laufpuffer wurde bei 50 Volt (45 mA) während 2,5 h durchgeführt; das Gel wurde getrocknet, gefolgt von Autoradiographie während 2 h bei -80°C unter Verwendung eines XAR Films (Kodak).

### Beispiel 5

### Transport von Transferrin-Polylysin-Konjugaten in lebende Zellen.

Um nachzuweisen, daß die im Beispiel 1 beschriebenen Transferrin-Polylysin-Konjugate effizient in lebende Erythroblasten aufgenommen werden, wurden FITC-markierte Konjugate verwendet. Es ist bekannt (Schmidt et al,1986), daß FITC- markiertes Transferrin nach einigen Stunden Inkubation mit Erythroblasten, denen vorher Transferrin entzogen worden war, in Vesikeln innerhalb der Zelle nachweisbar war (Untersuchung im Fluoreszenzmikroskop).

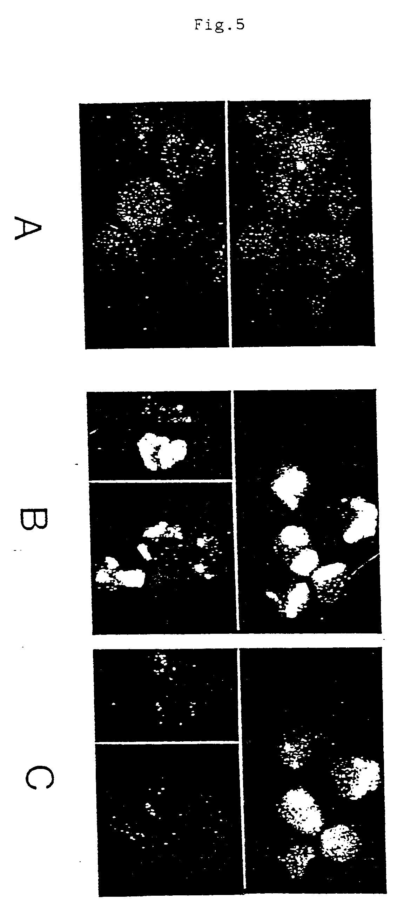

Im vorliegenden Beispiel wurden Erythroblasten (durch ein EGF-Rezeptor-Retrovirus transformiert, Khazaie et al,1988) 18 Stunden in transferrinfreiem Differenzierungsmedium (Zusammensetzung in Zenke et al,1988) bei 37°C (Zellkonzentration 1,5x10⁶/ml) inkubiert. Nach Zugabe der verschiedenen Transferrin-Polylysin-Konjugate (oder, als Kontrolle, der entsprechenden Menge sterilen aq bidest) wurden die Zellen bei 37°C in Gegenwart von 10 ng/ml EGF (um den transformierten Zustand aufrechzuerhalten) inkubiert. Nach 24 und 48 Stunden wurden ca. 5x10⁵ Zellen entnommen, 1 mal in phosphatgepufferter physiologischer Kochsalzlösung (PBS; pH 7.2) gewaschen, mit dem 5Ofachen Volumen einer Mischung von 3.7 % Formaldehyd und O.O2% Glutaraldehyd in PBS fixiert (10 min, 40°C), 1 mal in PBS gewaschen, in Elvanol eingebettet und im Fluoreszenzmikroskop (Zeiss Axiophot,Schmalband-FITC und TRITC-Anregung) untersucht. Gleichzeitig wurde in anderen Aliquots der verschiedenen Ansätze die Wachstumsrate der Zellen bestimmt. Hierzu wurden 1OO µl Zellsuspension entnommen und der Einbau von ³H-Thymidin (8 µCi/ml,2 Stunden) bestimmt, wie in Leutz et al,1984 beschrieben. Aus Fig. 5 geht hervor, daß die mit Transferrin-Polylysin inkubierten Erythroblasten nach 24 Stunden 2 bis 1O stark fluoreszierende Vesikel aufweisen, die in den Kontrollen nicht nachweisbar sind. Tabelle A zeigt, daß mit Ausnahme der Fraktion 6 alle Konjugate von praktisch allen Zellen aufgenommen worden sind.

Fig. 5 zeigt Fluoreszenzaufnahmen von Hühnererythroblasten, die 24 h ohne (A) oder mit FITC-markierten Transferrin-Polylysin-Konjugaten (B,C) inkubiert wurden. Bei Anregung mit blauem Licht (B, zum Nachweis von FITC) sind deutlich mehrere fluoreszierende Vesikel in jeder Zelle zu erkennen. Die Spezifität dieser Fluoreszenz wird dadurch gezeigt, daß die Vesikelfluoreszenz bei Anregung mit grünem Licht (C: bei welcher eine ähnliche unspezifische Fluoreszenz der Zellen wie in A zu sehen ist) nicht auftritt (C).

Die Tatsache, daß die Zellen in allen Proben gleich schnell wachsen (wie durch Einbau von tritiiertem Thymidin (³H TdR) gemessen, Tabelle A) beweist, daß die Zellen durch die Polylysin-Konstrukte nicht geschädigt werden und somit eine unspezifische Aufnahme (z.B. über durchlässig gewordene Zellmembranen) auszuschließen ist.

### Beispiel 6

Ziel der in diesem Beispiel durchgeführten Versuche war es zu zeigen, daß die hier verwendeten Transferrin-Polylysin-Konjugate von der Zelle wie natives Transferrin verwendet werden, d.h. den normalen Transferrinzyklus mit ähnlicher Effizienz durchlaufen. Als Testsystem hierfür sind Erythroblasten, die durch "Abschalten" des transformierenden Onkogens zur Ausreifung in normale Erythrozyten induziert werden können, besonders geeignet (Beug et al,1982). Aus der Literatur geht hervor, daß solche Zellen zur normalen Reifung hohe Konzentrationen an Transferrin-Eisen Komplex benötigen (1OO -2OO µg/ml, 3 fach geringere Konzentrationen verhindern das Ausreifen der Zellen und führen nach mehreren Tagen zum Tod der Zellen (Kowenz et al,1986)). Auch konnte gezeigt werden (Schmidt et al,1986), daß "recycling" ,also Wiederverwendung von Transferrinrezeptoren und damit ein mit optimaler Geschwindigkeit ablaufender Transferrinzyklus für eine normale in vitro Differenzierung unerläßlich ist.

Erythroblasten (durch das EGF-Rezeptor-Retrovirus transformiert) wurden durch Entzug von EGF und Zugabe einer optimalen Menge an partiell gereinigtem Hühner-Erythropoietins (Kowenz et al.,1986,frei von Transferrin) zur Differenzierung induziert. Die Inkubation erfolgte bei einer Zellkonzentration von 1x10⁶/ml in transferrinfreiem Differenzierungsmedium bei 42°C und 5% CO₂. Bei Inkubationsbeginn wurde entweder nativer Transferrin-Eisen-Komplex (Sigma, 100 µg/ml) oder die mit Eisen gesättigten Transferrin-Polylysin-Konjugate (Konzentration ebenfalls 1oo µg/ml zugegeben. Wachstum und Reifungszustand der Zellen wurden auf folgende Art nach 24 und 48 h analysiert:
1. Bestimmung der Zellzahl (im Coulter Counter, Modell ZM, Beug et al,1984)
2. Aufnahme von Zell-Größenverteilungen (im Coulter-Channelyzer Mod. 256) und
3. Photometrische Messung des Hämoglobingehalts der Zellen (Kowenz et al., 1986)

Außerdem wurden Aliquots der Ansätze nach 72 Stunden in einer Cytozentrifuge (Shandon) auf Objektträger zentrifugiert und einem histochemischen Nachweis für Hämoglobin unterzogen (Färbung mit neutralem Benzidin plus Diff-Quik Schnellfärbung für Blutzellen, Beug et al.1982).

Die Ergebnisse in Tabelle B zeigen deutlich, daß Zellen, die in Gegenwart der Polylysin-Transferrin Konjugate Fraktion 1 bis 5 zur Differenzierung induziert wurden, genauso effizient und mit gleicher Geschwindigkeit ausreifen wie solche, die mit nativem Transferrin-Eisen inkubiert wurden. Die Zellen in den transferrinfreien Kontrollen dagegen zeigten ein stark verlangsamtes Zellwachstum und akkumulierten nur geringe Mengen an Hämoglobin. Die Untersuchung des Zellphänotyps an gefärbten Cytospin-Präparaten ergab, daß die mit Polylysin-Transferrin-Konjugaten inkubierten Zellen genauso zu späten Retikulocyten (late reticulocytes,Beug et al.,1982) herangereift waren wie die mit nativem Transferrin behandelten, während die ohne Transferrin inkubierten Zellen eine Mischung aus desintegrierten und unreifen, erythroblastenähnlichen Zellen darstellten (Schmidt et al,1986). Nur die mit Transferrin-Polylysin-Fraktion 6 behandelten Zellen wiesen einen geringeren Hämoglobingehalt und einen größeren Prozentsatz an unreifen Zellen auf (Tabelle B). Dies zeigt, daß die mit besonders viel Polylysin konjugierte Fraktion 6 weniger gut im Transferrinzyklus funktioniert. Gleichzeitig weist dieses Ergebnis auf die Empfindlichkeit der Testmethode hin.

### Beispiel 7

In ähnlicher Weise wie in Beispiel 6 wurden verschiedene Transferrin-Polylysin-Konjugate und Transferrin-Protamin-Konjugate auf ihre Fähigkeit untersucht, bei der Reifung von Hühnererythroblasten zu Erythrozyten den nativen Transferrin-Eisenkomplex funktionell zu ersetzen.
Es wurde bereits gezeigt, daß terminal differenzierende Hühnererythroblasten einen optimal funktionierenden Transferrinzyklus verlangen; d.h. ohne Transferrin oder bei Hemmung des Transferrinrezeptor-Recycling sterben die Zellen ab (Kowenz et al., 1986; Schmidt et al., 1986). Da das normalerweise verwendete teilgereinigte Hühnererythropoietin noch Transferrin enthält, wurde EPO durch einen transferrinfreien, teilweise gereinigten erythroiden Wachstumsfaktor ersetzt, um die erythroide Differenzierung zu ermöglichen (REV-Faktor; Kowenz et al., 1986; Beug et al., 1982): Als Zielzellen wurden Erythroblasten, die mit einem den menschlichen epidermalen Wachstumsfaktor-Rezeptor (EGFR) zusammen mit einem temperatur-empfindlichen v-myb Oncogen enthaltenden Retrovirus transformiert waren in CFU-E Medium (Radke et al., 1982) in Anwesenheit von 20 ng/ml EGF vermehrt. Diese Zellen werden durch EGF zur abnormalen Vermehrung angeregt, Entzug des Wachstumsfaktors EGF bei gleichzeitiger Zugabe von REV-Faktor bewirkt, daß die Zellen in die normale Differenzierung eintreten. Nach zweimaligem Waschen in transferrinfreiem Medium wurden die Zellen in transferrinfreies Medium ausgesät und verschiedene Mengen von mit Eisen gesättigtem Transferrin oder Transferrin-Polykation-Konjugaten zugefügt (vor oder nach deren Komplexierung mit Plasmid DNA). Nach 1, 2 und 3 Tagen Inkubation bei 42°C wurde der Differenzierungszustand der Zellen durch Cytozentrifugation und histochemische Färbung oder durch quantitative Hämoglobinbestimmung festgestellt.

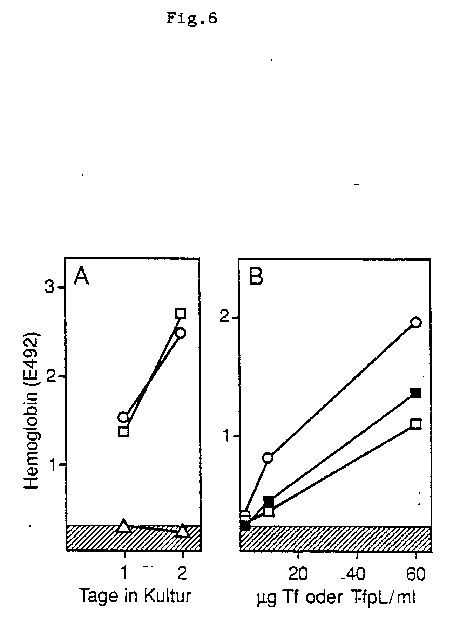

Das Ergebnis dieser Versuche ist in Fig.6 bzw. Tab.C dargestellt.

Die Zellen (1 x 10⁶/ml) wurden in conalbuminfreiem Differenzierungsmedium, (Zenke et al., 1988), ergänzt durch 1 µg/ml Insulin und REV-Faktor bei optimaler Konzentration (Kowenz et al., 1986; Verdünnung 1 : 5.000), einmal ohne Zusätze (Dreiecke), einmal mit eisengesättigtem Conalbumin (Kreise) und einmal mit eisengesättigtem TfpL 270-Konjugaten (je 100 µg/ml); Vierecke in 14 mm Schalen eingebracht. Nach Inkubation während 24 und 48 Stunden wurde der Hämoglobingehalt in 100 µl Aliquots photometrisch gemessen. Der schraffierte Bereich gibt den Hämoglobingehalt in Abwesenheit von Transferrin gezüchteter Zellen an (Mittelwert aus 4 Bestimmungen; Fig. 6A).

Um die erythroide Differenzierung als Funktion der Transferrin- bzw. Transferrin-Polylysin-Konzentration zu analysieren, wurden die Zellen, wie oben beschrieben, in Medium, enthaltend die angegebenen Mengen eisengesättigtes Conalbumin (offene Kreise), TfpL 90 (offene Vierecke) oder TfpL 270 (geschlossene Vierecke) eingebracht und nach 2 Tagen der Hämoglobingehalt photometrisch bestimmt (Fig. 6B).

### Tabelle C:

Die erythroide Differenzierung wurde verfolgt durch photometrische Hämoglobinbestimmung (vgl. Fig. 6), durch Auszählen in einen Coulter-Zähler oder durch Cytozentrifugation und anschließende neutrale Benzidinfärbung (zur Hämoglobinbestimmung) plus histologische Farbstoffe (Diff Quik; Beug et al., 1982b). Die Endkonzentrationen von Transferrin und Transferrin-Konjugaten betrugen in Versuch 1 60 µg/ml; in den Versuchen 2 und 3 100 µg/ml. Die DNA-Konzentration in Versuch 2 betrug 10 ug/ml. Der Anteil an desintegrierten Zellen, reifen Zellen (LR: späte Reticulozyten; E: Erythrozyten) und unreifen Zellen (Ebl) wurde nach den von Beug et al., 1982b und Schmidt et al., 1986 beschriebenen Methoden bestimmt. Die erhaltenen Resultate zeigen, daß zwei verschiedene Transferrin-Polylysin-Konjugate (TfpL₉₀ oder TfpL₂₇₀) ebenso wie das Transferrin-Protamin-Konjugat fähig sind, natives Transferrin funktionell zu ersetzen, indem sie einen raschen Transport von Eisen in differenzierende rote Zellen gewährleisteten, wobei ihre spezifische Aktivität um das 1,5 bis 2 fache niedriger war (vgl. Fig. 6). Die Komplexierung von DNA mit Transferrin-Polylysin 270 und Tranferrin-Protein veränderte die biologische Aktivität der Konjugate nicht wesentlich. In einem Kontrollexperiment wurde festgestellt, daß bei Zugabe von Polylysin oder Protamin, gemischt mit einer geeigneten Menge von Eisencitrat anstelle der Transferrin-Konjugate die Zellen nicht differenzieren konnten und abstarben, ähnlich wie bei Zellen in den Vergleichsproben, die ohne Transferrin inkubiert wurden.

Insgesamt haben die Versuche gemäß den Beispielen 6 und 7 gezeigt, daß beide Arten von Polykation-Transferrin-Konjugaten, Eisen nur geringfügig weniger effizient transportierten als natürliches Transferrin.

### Beispiel 8

### Polylysin-Transferrin-Konjugate ermöglichen die Aufnahme von DNA in Hühnererythroblasten.

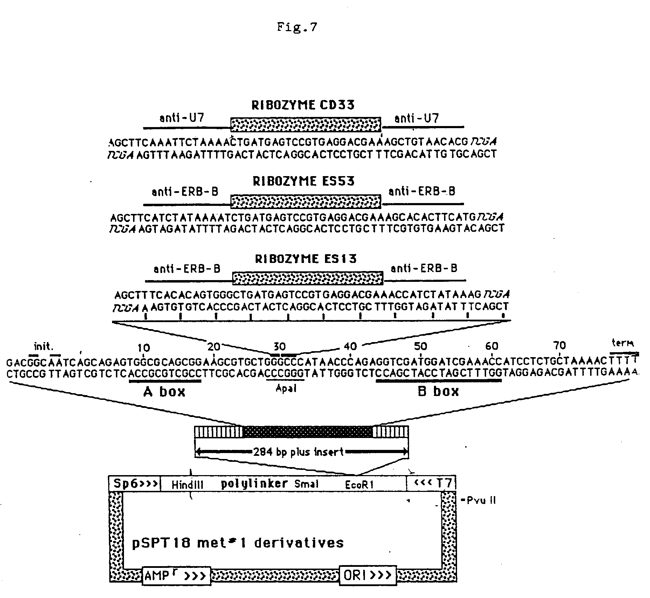

In vorliegendem Beispiel sollte untersucht werden, ob DNA in einer Größe, die der von tDNA-Ribozymen (vgl. Fig. 7) entspricht,von Transferrin-Polylysin-Konjugaten effizient in das Zellinnere transportiert werden kann. Im vorliegenden Beispiel wurde tDNA mit einem Insert der Sequenz 
CGTTAACAAGCTAACGTTGAGGGGCATGATATCGGGCC
CCGGGCAATTGTTCGATTGCAACTCCCCGTACTATAGC
 Molekulargewicht ca. 3OO.OOO, mit gamma 32P ATP endmarkiert (Maniatis), verwendet. Ungefähr O.3 µg dieser DNA, gelöst in 20 µl TE Puffer, wurden entweder mit 1O µg nativem Transferrin, mit 10 µg Transferrin-Polylysin Konjugat Fraktion 3,jeweils gelöst in 5o µl aq bidest plus 4oo µg/ml Rinderserumalbumin (Beug H., et al., 1982) oder mit 50 µl dieses Lösungsmittels ohne Transferrin vermischt. Die DNA-Proteinmischungen wurden zu je 2 ml transferrinfreiem Differenzierungsmedium zugegeben, 4x1o⁶ Hühnererythroblasten, (die mit einem EGF-Rezeptor-Retrovirus transformiert waren und 18Hin transferrinfreiem Medium in Gegenwart von EGF vorinkubiert wurden, (Khazaie K., et al.,1988) zugegeben und die Ansätze 8 Stunden bei 37°C und 5 % CO₂ inkubiert. Danach wurden die Zellen abzentrifugiert, der Überstand abgenommen und die Zellen 3 mal in transferrinfreiem Medium gewaschen. Zellsediment und Kulturmedium wurden in1% SDS,1mg/ml Proteinase K, 3OOmM NaCl, 2OmMTris pH 8.0, 1OmM EDTA (PK/SDS-Puffer) aufgenommen, 30 min bei 37°C inkubiert, mit Phenol/Cloroform extrahiert,und die DNA durch Ethanolfällung isoliert. Isolierte DNA mit einer Radioaktivität von ingesamt 2000 cpm wurde auf einem nicht-denaturierenden 3.5 % Acrylamidgel aufgetrennt (TBE, Maniatis) und die DNA durch Autoradiographie nachgewiesen. Es wurde gezeigt, daß in der mit Tranferrin-Polylysin behandelten Zellprobe etwa 5-10 mal mehr DNA von den Zellen aufgenommen worden ist als in den Kontrollproben mit nativem Transferrin.

### Beispiel 9

### Polylysin-Transferrin-Konjugate ermöglichen die Aufnahme und Expression von Plasmid-DNA in Hühnererythroblasten.

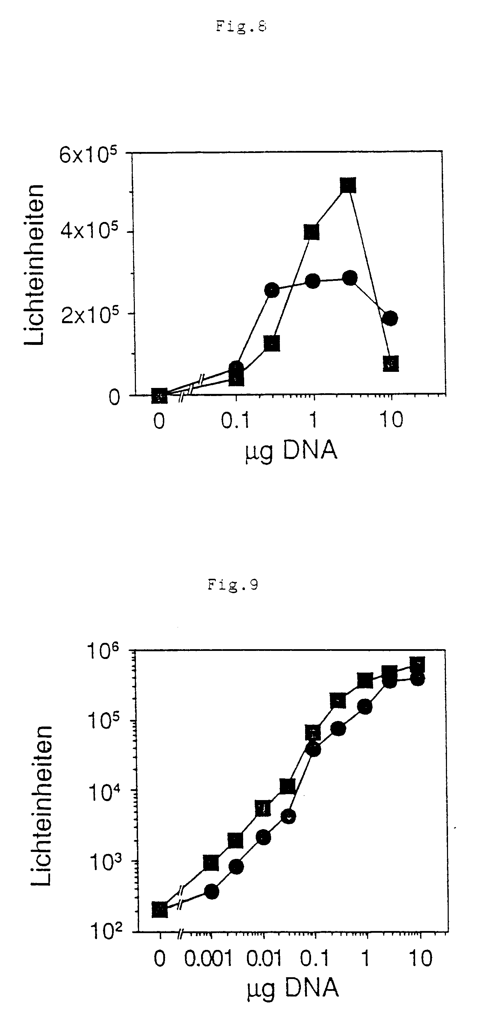

In diesen Versuchen wurde Plasmid-DNA, enthaltend das Photinus pyralis - Luciferase-Gen als Reporter-Gen, verwendet, um Gentransfer und Expression zu untersuchen. Dazu wurde pRSVluc Plasmid-DNA (De Wet, J.R., et al., 1987) unter Verwendung der Triton-X Lyse Standard-Methode (Maniatis) hergestellt ,gefolgt von CsCl/EtBr Gleichgewichtsdichtegradienten-Zentrifugation, Entfärbung mit Butanol-1 und Dialyse gegen 10 mM Tris/HCl pH 7,5, 1 mM EDTA. In einer typischen Komplexbildungsreaktion wurden 10 µg Transferrin-Polylysin- oder Transferrin-Protamin-Konjugate zu 3 µg pRSVluc Plasmid-DNA, enthalten in 250 µl 0,3 M NaCl, unter vorsichtigem Rühren langsam hinzugefügt (Es wurde festgestellt, daß bei Einhaltung dieser Bedingungen bis zu 100 µg Transferrin-Polykation-Konjugat und 30 µg Plasmid-DNA in einem Endvolumen von 500 µl verwendet werden können, ohne daß die Konjugat/DNA-Komplexe ausfallen). Nach 30 min bei Raumtemperatur wurden die Komplexe direkt zu 5 - 10 x 10⁶ HD3-Zellen (0,5 - 1 x 10⁶ Zellen pro ml, EBM₊H-Medium (Beug et al.,1982a; 37°C, 5 % CO₂) gefügt und 16 - 48 h lang inkubiert (als Zellinie wurde die ts-v-erbB transformierte Hühnererythroblastenzellinie HD3 verwendet). Die Zellen wurden geerntet (5 min bei 1500 x g, 4°C, zweimal mit Phosphat-gepufferter Salzlösung (PBS) gewaschen und in 100 µl 0,25 M Tris/HCl pH 7,5 aufgenommen. Zellextrakte wurden durch drei Zyklen Gefrieren und Auftauen, gefolgt von Hochgeschwindigkeitszentrifugation (15 min, 18.500 x g, 4°C) hergestellt. Aliquots solcher Zellextrakte wurden auf das Vorhandensein von Luciferaseenzymaktivität untersucht (De Wet, J.R., et al., 1987). Die Bioluminiszenz wurde unter Verwendung des Clinilumats (Berthold, Wildbach, BRD) gemessen.
Es wurde festgestellt, daß die Anwesenheit der Transferrin-Polykation/DNA-Komplexe im Kulturmedium keinen schädlichen Einfluß auf Zellwachstum und -vermehrung haben. Wie aus Fig. 8 ersichtlich ist, wurde maximale Luciferase-Aktivität erreicht, wenn 3 µg DNA/10 µg TfpL und 0,3 - 1 µg DNA/Tfprot verwendet werden. Unter der Annahme, daß alle gebildeten Konjugat/DNA-Komplexe identisch waren, entspricht das einem molaren Verhältnis von 25 bzw. 75 Konjugatmolekülen pro Molekül Plasmid-DNA. Daraus kann geschlossen werden, daß die DNA im Komplex zur Gänze durch die Konjugatmoleküle abgedeckt ist, und zwar bei einem Konjugat/DNA-Verhältnis, das offensichtlich Elektroneutralität (berechnet auf Basis der positiven Ladungen im Polykation, die erforderlich sind, die negativen Ladungen der Phosphatgruppen in der DNA zu neutralisieren) gewährleistet. Diese Annahme stimmt mit der Beobachtung überein, daß im Vergleich zu Transferrin-Polylysin dreimal mehr an weniger stark positiv geladenem Transferrin/Protamin für optimale Komplexbildung und Gentransfer benötigt wird. Diese Annahme stimmt auch mit dem in Beispiel 4 erhaltenen Ergebnis für das Konjugat/DNA-Verhältnis überein, das für effiziente Komplexbildung erforderlich ist.

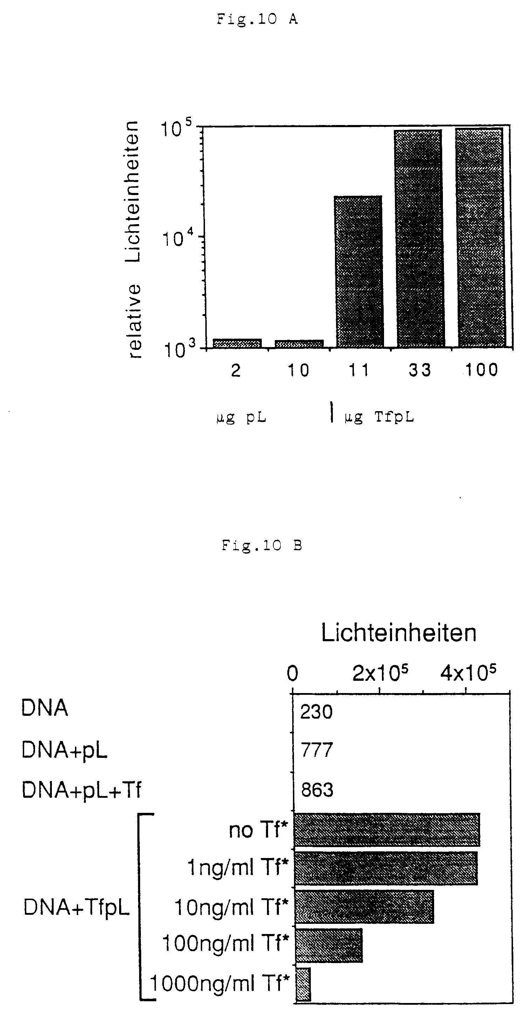

Unter Verwendung eines derart optimierten TfpL/DNA-Verhältnisses für die Komplexbildung wurde die Empfindlichkeit dieses Gentransfer-Systems bestimmt. Das Ergebnis dieser Versuche ist in Fig. 9 gezeigt: Weniger als 1 ng für Luciferase kodierende Plasmid-DNA zeigt noch ein nachweisbares Signal. Die Anwendung von mehr als 2 µg Plasmid-DNA, komplexiert mit 6 µg TfpL bzw. 20 µg Tfprot führt zu keiner weiteren Zunahme der Luciferaseaktivität, vermutlich aufgrund der Sättigung des Systems. Es wurde weiters festgestellt, daß keine besondere Salz- oder Ionenkonzentration für die Komplexbildung erforderlich ist, weil TfpL/DNA-Komplexe, die bei verschiedenen Salzkonzentrationen gebildet wurden (0, 20, 50, 100, 200 mM NaCl) in Gentransfer-Experimenten gleichermaßen wirksam waren. (Fig.8 und Fig.9: Kreise Tfprot, Vierecke TfpL). Es konnte gezeigt werden, daß die Aufnahme der Transferrin-Polykation/DNA-Komplexe in die Zelle über den Transferrin-Rezeptor abläuft. Zunächst, wie in Fig. 10A dargestellt ist, wurde festgestellt, daß die durch TfpL-DNA-Komplexe erreichte Luciferaseaktivität mindestens 100 mal höher ist als die Aktivität, die für pL-DNA-Komplexe gemessen wurde. Ein Vergleichsversuch ergab, daß ein Gemisch von Polylysin und Transferrin allein die Aufnahme von Plasmid-DNA nicht erleichtert. In einem weiteren Versuch wurden zu einer konstanten Menge TfpL-DNA-Komplex ein Überschuß an nativem Transferrin gefügt. Fig. 10B zeigt, daß freies Transferrin im Medium effizient um die durch TfpL vermittelte DNA-Aufnahme konkurriert, was in einer Verringerung der Luciferase-Enzymaktivität resultiert. Daraus läßt sich schließen, daß die Aufnahme der TfpL-DNA-Komplexe durch die Zelle über den Transferrin-Rezeptor erfolgt.

### Beispiel 10

In Vorversuchen war anhand der Transfektion von Hühnerfibroblasten mit erbBschnitt DNA festgestellt worden, daß die erbBschnitt-Ribozym-tDNA in Hühnerzellen exprimiert wird.

Mit diesem Beispiel konnte gezeigt werden, daß tDNA-Ribozyme,die gegen das erb B Onkogen gerichtet sind, mit Hilfe von Polylysin-Transferrin-Konjugaten in erb B-transformierte Hühnererythroblasten eingebracht werden und die transformierende Wirkung des Onkogens schwächen können.

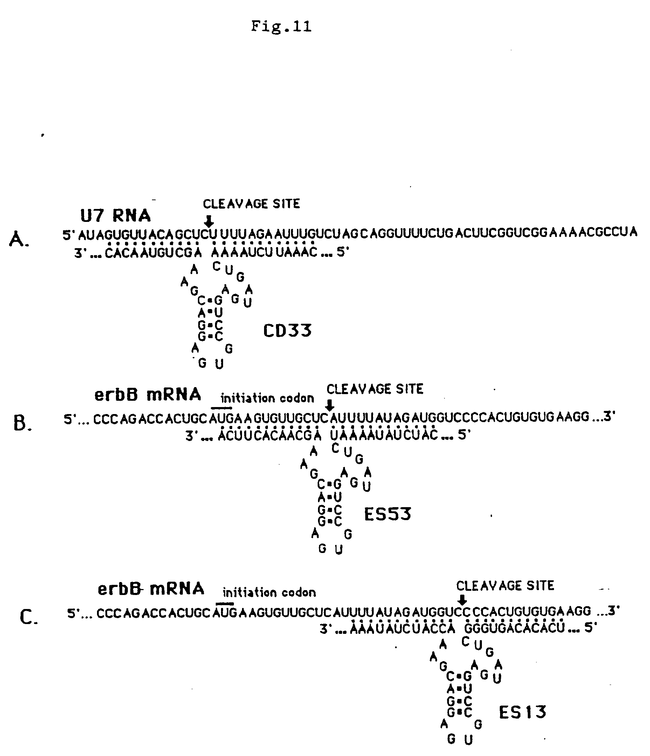

Zwei tRNA-Ribozymgene, gerichtet gegen die Translationsinitions-Region von erbB wurden, konstruiert (vgl. Fig. 7 und 11). Ca. 100 µg jedes das Gen enthaltenden Plasmids wurden EcoRI verdaut, um das tRNA-Ribozymgen auf einem 325 bp-Fragment freizusetzen.

Die Verdauprodukte wurden mittels Klenow-Fragment endmarkiert und mittels Gelelektrophorese durch ein 2% Agarose/TBE-Gel gereinigt. Das Vektorfragment und die tRNA-Ribozymgen-Fragmente wurden durch Ethidiumbromid-Färbung lokalisiert, ausgeschnitten und durch Elektroelution, Phenol/Chloroform -und Chloroformextraktion und Ethanolfällung gewonnen. Die gereinigten, radioaktiv markierten DNA-Fragmente wurden daraufhin unter Benutzung des Transferrin-Polylysin-Transportsystems dazu verwendet, die Aufnahme und die Hemmung der erbB-RNA zu bestimmen. Als Kontroll-DNA wurde der Vektor pSPT 18 verwendet.

Als Testzellsystem wurde eine Hühnererythroblastenzellinie gewählt, die durch eine temperatursensitve Mutante (ts 34,Graf et al 1978) des Vogel- Erythroblastosevirus AEV transformiert ist (Beug et al, 1982 b). Das in diesen Zellen ebenfalls exprimierte erb A Onkogen kann durch einen spezifischen Proteinkinase-Hemmer (H 7) inhibiert werden. (Es wurde festgestellt, daß das v-erbA-Onkogen in vivo und in vitro (d.h. als bakteriell exprimiertes Protein) an zwei Stellen, nämlich Ser 28 und Ser29, durch Proteinkinase C bzw. durch cAMP-abhängige Proteinkinase phosphoryliert wird. Mutation dieser Serine zu Alaninen verhindert die Phosphorylierung und zerstört die v-erbA-Onkogenaktivität. H7 ist ein spezifischer Inhibitor dieser beiden Kinasen und ist in der Lage, in v-erbA-v-erbB haltigen Erythroblasten selektiv die durch v-erbA verursachten Veränderungen (z.B. Blockierung der Differenzierung) aufzuheben.)

Es ist bekannt, daß Erythroblasten deren erbB Onkogen inaktiviert wird - z.B. durch Erhöhung der Temperatur im Falle einer temperaturempfindlichen erb B Mutante - dazu induziert werden, zu Erythrozyten auszureifen.

Eines der ersten Anzeichen für diesen Vorgang ist eine Induktion der Hämoglobinsynthese, die durch einen empfindlichen Nachweis (saure Benzidinfärbung, Orkin et al, 1975, Graf et al,1978) auf dem Niveau der Einzelzelle nachgewiesen werden kann. Als phänotypische Wirkung eines gegen erb B gerichteten Ribozyms in diesem Testsystem wäre somit eine spezifische Erhöhung der Zahl benzidin-positiver Zellen zu erwarten.

Die diesem Beispiel zugrundeliegende Versuchsreihe wurde folgendermaßen durchgeführt: Die verschiedenen DNA -Präparationen (siehe oben und Tabelle D),gelöst in 30 µl TE- puffer, wurden mit jeweils 1O µg nativem Transferrin -Eisenkomplex oder Transferrin-Polylysin -Konjugat (gelöst in 50 µl aq. bidest.) gemischt und 30 min bei 37°C inkubiert.

Im Falle der Vektor DNA, (1O µg) wurde entprechend mehr (100 µg) der Transferrin-Präparationen verwendet. Die DNA-Transferrin-DNA-Mischungen wurden zu je 1 ml transferrinfreiem Differenzierungsmedium (Zenke et al., 1988) zugegeben. Die Testzellen (pro Ansatz 3x10⁶) wurden vor dem Versuch 60 min in transferrinfreiem Differenzierungsmedium bei 42°C inkubiert, (hierdurch wird die Transferrinaufnahme verstärkt) und zu den DNA-Transferrin-haltigen Ansätzen hinzugegeben. Nach 6 h, 18 h und 68 h (Behandlung der Zellen siehe unten) wurden Proben entnommen, wie beschrieben, in Überstand und Zellsediment aufgetrennt, in PK/SDS Puffer aufgenommen und die DNA analysiert.

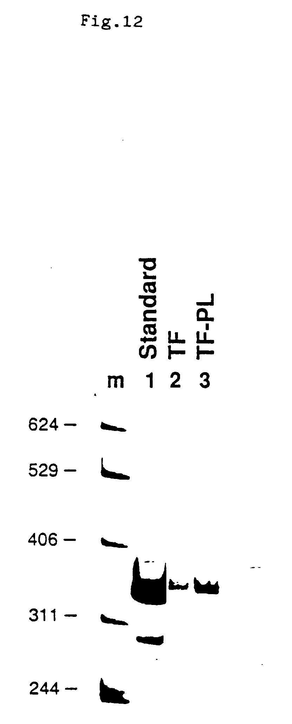

Fig. 12 zeigt, daß, analog Beispiel 8, in der mit Transferrin-Polylysin behandelten Zellprobe etwa 5-10 mal mehr DNA von den Zellen aufgenommen wurde als in den Kontrollproben mit nativem Transferrin.
Spur m: Molekulargewichtsmarker: pBR322 DNA, gespalten mit HpaII und unter Verwendung des Klenow-Fragments der DNA-Polymerase mit alpha-³²P-CTP radioaktiv markiert (Maniatis)
Spur 1: 2000 cpm ES13-Fragment
Spur 2: Material von Zellen, behandelt mit Transferrin und ES13
Spur 3: Material aus Zellen, behandelt mit Transferrin-Polylysin und ES13

Nach Inkubationsende (6 h) wurden die Zellen abzentrifugiert und in transferrinhaltigem Differenzierungsmedium mit Erythropoietin und Insulin (Kowenz et al,1986, Zenke et al 1988, 2 ml pro Ansatz und bei 37°C d.h. in Anwesenheit eines aktiven v-erb B-Proteins) weitere 72 Stunden inkubiert.

Folgende Ergebnisse wurden erhalten:
1. Ähnlich wie in Beispiel 8 konnte eine verstärkte Aufnahme von DNA in der Größe der erbBschnitt DNAs in den mit Transferrin-Polylysin behandelten Zellproben (etwa 5 fach) beobachtet werden.
2. Tabelle D zeigt, daß in allen Fällen, in denen erbBschnitt-Ribozym-tDNA mit Hilfe von Polylysin-Transferrin-Konstrukten in erb B transformierte Erythroblasten eingebracht wurde, der Prozentsatz an Benzidin-positiven Zellen signifikant (auf ca. das Doppelte) erhöht war (als Referenz dienten die Proben, die mit Vektor-DNA behandelt wurden und in denen die Verwendung von Polylysin-Transferrin-Konjugaten erwartungsgemäß nicht zu einer Erhöhung der Zahl benzidin-positiver Zellen führte).

### Beispiel 11

### Effiziente Bindung und Internalisierung von Transferrin-Polylysin/DNA-Komplexen in hämatopoietischen Hühnerzellen

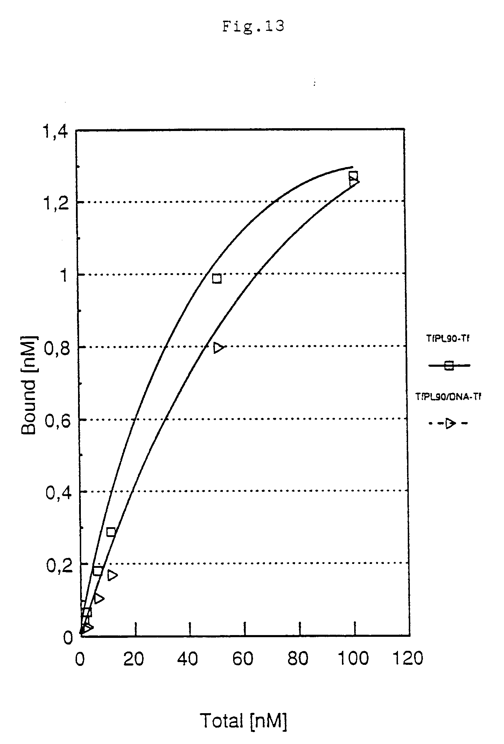

Die Bindung von TfpL und TfpL-DNA an Zelloberflächenrezeptoren wurde mit Tritium-markierten Substanzen nach der von Stein et al., 1984, beschriebenen Methode gemessen. ³H-markiertes TfpL wurde durch Konjugation von markiertem Polylysin mit Transferrin nach der in Beispiel 1 beschriebenen Methode hergestellt. Die Markierung von Polylysin 90 wurde durch Behandlung mit Formaldehyd und ³H-markierten Natriumborhydrid durchgeführt (Ascoli und Puet, 1974). Das Ergebnis dieser Versuche ist in Fig. 13 dargestellt. Markiertes TfpL 90 (Vierecke) oder markiertes TfpL 90, komplexiert mit pB-SK⁻-DNA (Promega Biotech, hergestellt mittels Triton-X-Lyse, CSCl/EtBr-Gleichgewichts-Dichtegradientenzentrifugation, Entfärbung mit 1-Butanol und Dialyse gegen 10mM Tris/HCl pH 7,5, 1mM EDTA)(Dreiecke) wurden auf ihre spezifische Bindung an den Transferrin-Rezeptor von HD3-Zellen untersucht. Dazu wurden die Konjugate bzw. Komplexe (0,1 - 100 nM) zu HD3-Zellen (1 x 10⁶/ml in MEM (= Eagle's Minimum Medium)+ 1%BSA) gefügt und 3 h inkubiert. Fig.13 zeigt, daß sowohl die Konjugate als auch die Komplexe an HD3-Zellen derart binden, daß Sättigung eintritt. Die aus diesen Daten errechneten scheinbaren Bindungskonstanten betrugen 22 nM für TfpL und 43 nM für TfpL-DNA-Komplexe. Obwohl etwas höher, stimmen diese Werte relativ gut mit denen für natives Transferrin, die mit 15 nM bestimmt worden waren, überein.

Um die Aufnahme von TfpL-DNA-Komplexen in intrazelluläre Vesikel zu verfolgen, wurden zunächst HD3-Zellen mit Transferrin-freiem Differenzierungsmedium bei 37°C 18 h lang inkubiert. Nach Zusatz von FITC-Transferrin bzw. von TfpL-Konjugaten (mit FITC an der Polylysin-Gruppe markiert, in einigen Experimenten mit DNA komplexiert), wurden die Zellen weitere 18 h inkubiert. Die Zellen wurden cytozentrifugiert, mit einer Mischung aus 3,7 %igem Formaldehyd und 0,02 %igem Glutaraldehyd fixiert, mit PBS gewaschen, in Mowiol 4.88 eingebettet und in einem Zeiss Axiophot Fluoreszenzmikroskop untersucht. Als Kontrolle wurde FITC-markierter Ziegen-Anti-Mausantikörper (0,1 mg/ml) verwendet (vgl. Beispiel 5). Für die quantitative Bestimmung von FITC-Tf, FITC-TfpL bzw. FITC-TfpL/DNA wurden die Zellen 6 h lang mit dem jeweiligen Präparat (Tf: 40 µg/ml; TfpL270: 50 µg/ml; Tfpl270 plus pB-SK⁻DNA (Promega Biotech, hergestellt mittels Triton-X-Lyse, CSCl/EtBr-Gleichgewichts-Dichtegradientenzentrifugation, Entfärbung mit 1-Butanol und Dialyse gegen 10mM Tris/HCl pH 7,5, 1mM EDTA): 50 µg/ml bzw. 16 µg/ml; Bindungspuffer) inkubiert, 3 x in kaltem PBS/BSA gewaschen und der quantitativen FACS-Analyse in einem Becton-Dickinson (BD) FACSAN unterworfen.

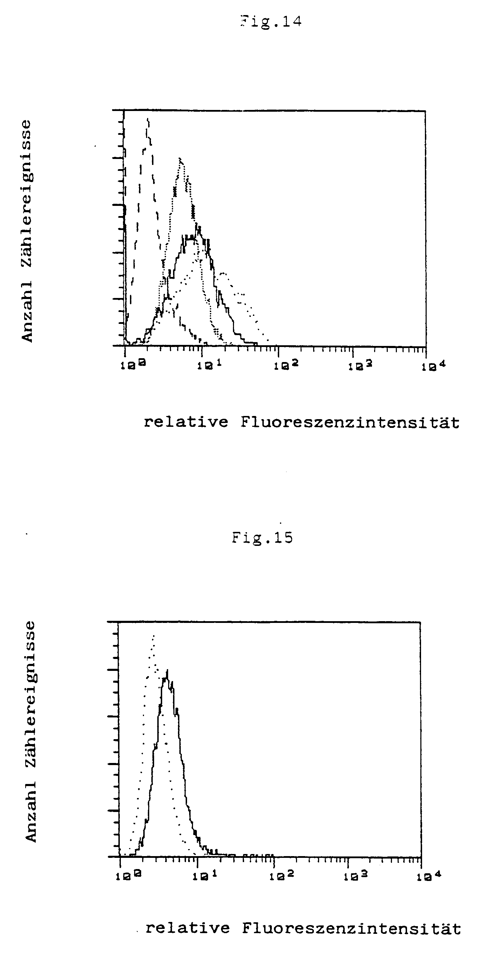

Fig. 14 zeigt, daß sowohl mit TfpL als auch mit TfpL-DNA alle Zellen eine mehr als 10 x erhöhte relative Fluoreszenz aufweisen, was darauf hinweist, daß die Konjugate bzw. Komplexe von mehr als 95 % der Zellen aufgenommen werden (Tf:.....; TfpL:. . . ; TfpL/DNA: ; Bindungspuffer: - - -).

### Beispiel 12

### Expression von DNA, die über TfpL in die Zelle aufgenommen wurde

Nachdem in den vorangegangenen Beispielen festgestellt worden war, daß der Gentransfer mit TfpL nicht schädlich für das Zellwachstum ist, wurde die Wirkung von TfpL-DNA-Komplexen getestet, die über einen längeren Zeitraum auf Zellen angewendet wurden (als DNA wurde Plasmid-DNA, enthaltend das Luciferase-Gen, wie im Beispiel 9 beschrieben, verwendet). In diesem Versuch wurde jeweils dieselbe Konzentration von HD3-Zellen 1 bis 4 Tage lang mit oder ohne tägliche Ergänzung von TfpL-DNA-Komplexen inkubiert.

In verschiedenen zeitlichen Abständen wurden Aliquots auf Luciferase-Enzym-Aktivität untersucht, wie in Beispiel 9 beschrieben. In den Kulturen mit wiederholter Zugabe der Komplexe wurde ein relativ hohes Maß an Luciferase-Gen Expression gemessen (100.000 bis 200.000 Lichteinheiten/10⁷ Zellen), das während des gesamten Untersuchungszeitraums im wesentlichen konstant blieb. Während dieses Zeitraums wurden keine cytotoxischen Wirkungen beobachtet. Wenn Zellen nur einmal mit den Komplexen beladen wurden, nahm die Luciferase-Aktivität zwischen dem 2. und 4. Tag um das 10 bis 20fache ab. Diese Ergebnisse zeigen, daß trotz offensichtlich vorübergehender Expression des Luciferase-Gens, das mit Hilfe der erfindungsgemäßen Konjugate in die Zelle eingeführt wurde, durch wiederholte Zugabe eine konstant hohe Expression der eingeführten Gene aufrechterhalten werden kann.

### Beispiel 13

Um festzustellen, wie groß der Anteil von Zellen ist, die tatsächlich Plasmid-DNA exprimieren, die über Transferrinfektion eingeführt wurde, wurden HD3-Zellen, wie in den vorangegangenen Beispielen beschrieben, mit TfpL/DNA-Komplexen inkubiert. Als DNA wurde pRSV-βGal-Plasmid-DNA( ) verwendet. Die Expression dieses Reporter-Gens wurde daraufhin in einzelnen Zellen mittels FACS-Analyse (Nolan et al., 1986) untersucht. Als Kontrolle wurde TfpL-pB-SK⁻-DNA(. . . ) verwendet. Dazu wurde das fluoreszierende βGal-Substrat FDG (Fluorescein-Di-β-d-Galaktopyranosid) mittels osmotischem Schock eingeführt und die Verteilung von Zellen, die Fluorescein enthalten, das aufgrund von βGal-Enzymaktivität aus FDG freigesetzt wurde, untersucht. Die gleichmäßige Verteilung Fluorescein enthaltender Zellen läßt den Schluß zu, daß ein großer Anteil von Zellen das βGal-Reporter-Gen exprimiert. Das Ergebnis dieser Versuche wird in Fig.15 gezeigt.

### Literatur

Ascoli, M. und Puett, D. (1974), Biochem.Biophys. Acta 371, 203-210.
Beug, H., et al., (1982a), J. Cell Physiol. Suppl. 1, 195-207.
Beug, H., et al., (1982b), Cell 28, 907-919.
Beug, H., et al. (1984), Cell 36, 963-972.
Cormier et al., (1988), Nucleic Acids Res. 16, 4583.
Deakin, H., et al., (1963), Biochem.J. 89, 296.
Graf et al., (1978), Nature 275, 496-501.
Huebers et al., (1987), Physiol.Rev. 67, 520-582.
Jung et al., (1981), Biochem.Res.Commun. 101, 599.
Kahazaie, K., et al., (1988), EMBO J., 10, 3061-3071.
Kowenz, E., et al., (1986), Mod.Trends in Human Leukemia VII, Springer Verlag, pp. 199-209.
Lemaitre et al., (1987), Proc.Natl.Acad.Sci. 84, 648.
Maniatis et al., Molecular Cloning, Cold Spring Harbor 1982.
Morvan et al., (1988) Nucleic Acids Res. 16, 833.
Nolan, G.P., et al., (1986), Proc.Natl.Acad.Sci. 85, 2603-2607.
Praseuth et al., (1988), Proc.Natl.Acad.Sci. 85, 1349.
Orkin, et al., (1975), Proc.Natl.Acad.Sci. 72, 98-102.
Radke, K., et al., (1982), Cell 31, 643-653.
Schmidt et al., (1986), Cell 46, 41-51.
Smith et al., (1986), Proc.Natl.Acad.Sci. 83, 2787.
Stavridis, J.C. and Psallidopoulos, M., 1982, Cell. Mol. Biol. 28(1), 15-18.
Stein et al., (1984), J. Biol.Chem. 259, 14762-14772.
Stein et al., (1988), Nucleic Acids Res. 16, 3209.
Stirchak et al., (1987), J.Org.Chem. 52, 4203.
Wu, G.Y.,et al., (1988), J.Biol.Chem. 263, 14621-14624.
Zamecnik et al. (1986), Proc.Natl.Acad.Sci. 83, 4143.
Zenke, M., et al., (1988), Cell 52, 107-119.

**Tabelle A**

| **Transport von PolyLysin-Transferrin in Erythroblasten** | | | | | |
|---|---|---|---|---|---|
| Ansatz | Medium | Transferrin-polyLysin | Vesikelfluoreszenz | | Viabilität (3H Td R-Einbau) |
| | | | 24 h | 48 h | 48 h |
| 1 | 2 ml | ohne Zusatz | <1 % | <1 % | 140.000 cpm |
| 2 | 2 ml | 145 µl H₂O | <1 % | <1 % | 126.000 cpm |
| 3 | 2 ml | 145 µl TfpL Fr1 | >90 %++ | >90 %++ | 137.000 cpm |
| 4 | 2 ml | 145 µl TfpL Fr2 | >90 %+++ | >90 %+++ | 161.000 cpm |
| 5 | 2 ml | 145 µl TfpL Fr3 | >90 %+++ | >90 %+++ | 153.000 cpm |
| 6 | 2 ml | 145 µl TfpL Fr4 | ca.80 %+++ | >90 %+++ | 151.000 cpm |
| 7 | 2 ml | 145 µl TfpL Fr5 | ca.60 %+++ | >90 %+++ | 153.000 cpm |
| 8 | 2 ml | 145 µl TfpL Fr6 | ca.40 %+++ | >90 %+++ | 165.000 cpm |
| ++ und +++ bezeichnen die relative Stärke der Vesikelfluoreszenz | | | | | |

**Tabelle B**

| **PolyLysin-Transferrin kann bei der Stimulierung der in-vitro induzierten Reifung** von **Erythroblasten normales Transferrin funktionell ersetzen** | | | | | | | |
|---|---|---|---|---|---|---|---|
| Nr. | Medium | Zusatz^{b} | Zellzahl (x 10⁶/ml) | | Hämoglobin E 492 | | Reifungsgrad % Reticulozyten |
| | | | 24 h | 48 h | 24 h | 48 h | 72 h |
| 1 | 2 ml | Fe-Transferrin | 3,28 | 4,38 | 1,38 | 2,74 | >80 % |
| 2 | 2 ml | - | 2,62 | 2,56 | 0,35 | 0,23 | <1 % |
| 3 | 2 ml | H₂0 | 2,60 | 2,42 | 0,30 | 0,13 | <1 % |
| 4 | 2 ml | TfpL Fr1 | 3,70 | 4,44 | 1,36 | 2,69 | >80 % |
| 5 | 2 ml | TfpL Fr2 | 3,56 | 4,24 | 1,16 | 2,51 | n.b.^{a} |
| 6 | 2 ml | TfpL Fr3 | 3,72 | 4,54 | 1,58 | 2,54 | >80 % |
| 7 | 2 ml | TfpL Fr4 | 3,48 | 4,56 | 1,57 | 2,55 | n.b. |
| 8 | 2 ml | TfpL Fr5 | 3,36 | 4,26 | 1,41 | 2,47 | n.b. |
| 9 | 2 ml | TfpL Fr6 | 3,58 | 4,4 | 1,14 | 1,93 | 60-65 % |

| | | | | | | | |
|---|---|---|---|---|---|---|---|
| ^{a}: nicht bestimmt | | | | | | | |
| ^{b}: Fe-Transferrin, 200 µg in 13 µl; TfpL-Fraktionen, 200 µg in 130 µl; H₂0, 130 µl | | | | | | | |

**Tabelle C**

| | Medium-Zusatz | | | | Differenzierungsparameter | | | |
|---|---|---|---|---|---|---|---|---|
| Versuch | Transferrin | Transferrin Konjugate | DNA | Zellzahl (x10⁶/ml) | Hb (E⁴⁹²) | % desint. Zellen | % LR+E | % Ebl |
| 1 | - | - | - | 2.56 | 0.259 | 56 | <1 | 44 |
| | + | - | - | 3.72 | 1.997 | 3 | 73 | 1 |
| | - | Tfpl90 | - | 3.67 | 1.105 | 5 | 54 | 8 |
| | - | Tfpl270 | - | 3.30 | 1.366 | 11 | 60 | 4 |
| 2 | - | - | pRSVLuc | 1.24 | 0.28 | | n.n. | |
| | + | - | pRSVLuc | 5.22 | 2.459 | | n.n. | |
| | - | Tfpl90 | pRSVLuc | 4.46 | 2.265 | | n.n. | |
| 3 | - | - | - | 2.1 | 0.222 | 79 | <1 | 21 |
| | + | - | - | 2.55 | 1.369 | 6 | 72 | 0 |
| | - | Tfpl90 | - | 2.64 | 1.016 | 10 | 56 | 7 |
| | - | Tf-Prot | - | 2.76 | 1.055 | 9 | 72 | 4 |

**Tabelle D**

| **Reifung (Hämoglobingehalt) von v-erbB - transformierten Erythroblasten, die v-erbB - Ribozym - DNA aufgenommen haben** | | | | | | | |
|---|---|---|---|---|---|---|---|
| Nr. | DNA | | | Transferrin | | Hämoglobin-Gehalt (% positiv nach saurer Benzidinfärbung) | |
| | Art | MW | Menge | Art | Menge | 14 h | 62 h |
| 1 | erbschnitt 13 | 2x10⁵ | 1 µg | Tf | 10 µg | <1 | 15+-3^{a} (3)^{b} |
| 2 | erbschnitt 13 | | | TfpL Fr5 | 10 µg | <1 | 37+-4 (2) |
| 3 | erbschnitt 53 | 2x10⁵ | 1 µg | Tf | 10 µg | <1 | 25+-2 (2) |
| 4 | erbschnitt 53 | | | TfpL Fr5 | 10 µg | <1 | 42+-1 (2) |
| 5 | vector ohne Ribozym | 2x10⁶ | 10 µg | Tf | 100 µg | <1 | 23+-3 (2) |
| 6 | vector ohne Ribozym | | 10 µg | TfpL Fr5 | 100 µg | <1 | 22+-2 (2) |
| 7 | erbschnitt 13 + 53 | s.o. | 0,5+0,5 µg | Tf | 10 µg | <1 | 21+-2 (2) |
| 8 | erbschnitt 13 + 53 | | 0,5+0,5 µg | TfpL Fr5 | 10 µg | <1 | 38+-2 (2) |

| | | | | | | | |
|---|---|---|---|---|---|---|---|
| a: Pro Bestimmung wurden > 200 Zellen ausgezählt, Werte +-Standardabweichung | | | | | | | |
| b: Anzahl unabhängiger Bestimmungen | | | | | | | |

## Patentansprüche

1. Konjugat, bestehend aus Transferrin und einem daran kovalent gekoppelten Polykation, dadurch gekennzeichnet, daß das Polykation ein synthetisches homologes Polypeptid oder ein Polyethylenimin ist.

2. Konjugat nach Anspruch 1, dadurch gekennzeichnet, daß das synthetische homologe Polypeptid ein Polylysin ist.

3. Konjugat nach Anspruch 1 oder 2, dadurch gekennzeichnet, daß das Polykation etwa 20 bis etwa 500 positive Ladungen aufweist.

4. Transferrin-Polykation/Nukleinsäure-Komplex, der über Transferrin-Rezeptor-vermittelte Endozytose in die menschliche oder tierische Zelle aufgenommen wird, enthaltend ein Konjugat nach einem der Ansprüche 1 bis 3.

5. Komplex nach Anspruch 4, dadurch gekennzeichnet, daß er als Nukleinsäure eine zur spezifischen Inhibierung von Genen bzw. der RNA-Funktion befähigte Nukleinsäure enthält.

6. Komplex nach Anspruch 5, dadurch gekennzeichnet, daß er eine Nukleinsäure enthält, die zur Inhibierung viraler Nukleinsäure befähigt ist.

7. Komplex nach Anspruch 5, dadurch gekennzeichnet, daß er eine Nukleinsäure enthält, die zur Inhibierung von Oncogenen oder anderen Schlüsselgenen, die Wachstum und/oder Differenzierung von Zellen kontrollieren, befähigt ist.

8. Komplex nach einem der Ansprüche 5 bis 7, dadurch gekennzeichnet, daß er als Nukleinsäure ein Ribozym, gegebenenfalls zusammen mit einer Carrier-RNA, bzw. das dafür kodierende Gen enthält.

9. Komplex nach Anspruch 8, dadurch gekennzeichnet, daß er als Nukleinsäure eine genetische Einheit, bestehend aus einem tRNA-Gen als Carrier-Gen und einem innerhalb dieses Gens angeordneten Ribozymgen, enthält.

10. Komplex nach einem der Ansprüche 5 bis 7, dadurch gekennzeichnet, daß er als Nukleinsäure ein Antisense-Oligonukleotid, gegebenenfalls zusammen mit einer Carrier-Nukleinsäure, bzw. im Falle eines RNA-Oligonukleotids das dafür kodierende Gen enthält.

11. Komplex nach Anspruch 4, dadurch gekennzeichnet, daß er eine für ein Protein kodierende Nukleinsäure enthält.

12. Verfahren zum Einführen von Nukleinsäure(n) in menschliche oder tierische Zellen in vitro mittels Transferrinrezeptor-vermittelter Endozytose, wobei man aus Transferrin-Polykation-Konjugat und Nukleinsäure(n) einen, vorzugsweise unter physiologischen Bedingungen löslichen, Komplex bildet und die Zellen mit diesem Komplex in Berührung bringt, dadurch gekennzeichnet, daß man ein in Anspruch 1 definiertes Konjugat einsetzt.

13. Pharmazeutische Zubereitung, enthaltend einen oder mehrere der in den Ansprüchen 4 bis 11 definierten Komplexe.

## Claims

1. Conjugate consisting of transferrin and a polycation covalently bound thereto, characterised in that the polycation is a synthetic homologous polypeptide or a polyethyleneimine.

2. Conjugate according to claim 1, characterised in that the synthetic homologous polypeptide is a polylysine.

3. Conjugates according to claim 1 or 2, characterised in that the polycation contains about 20 to about 500 positive charges.

4. Transferrin-polycation/nucleic acid complex which is absorbed into the human or animal cell by transferrin receptor-mediated endocytosis, containing a conjugate according to one of claims 1 to 3.

5. Complex according to claim 4, characterised in that it contains as nucleic acid a nucleic acid capable of specifically inhibiting genes or RNA function.

6. Complex according to claim 5, characterised in that it contains a nucleic acid capable of inhibiting viral nucleic acid.

7. Complex according to claim 5, characterised in that it contains a nucleic acid capable of inhibiting oncogenes or other key genes which control cell growth and/or differentiation.

8. Complex according to one of claims 5 to 7, characterised in that it contains as nucleic acid a ribozyme, optionally together with a carrier RNA, or the gene coding for it.

9. Complex according to claim 8, characterised in that it contains as nucleic acid a genetic unit consisting of a tRNA gene as carrier gene and a ribozyme gene arranged inside this gene.

10. Complex according to one of claims 5 to 7, characterised in that it contains as nucleic acid an antisense-oligonucleotide, optionally together with a carrier nucleic acid, or in the case of an RNA oligonucleotide, the gene coding therefor.

11. Complex according to claim 4, characterised in that it contains a nucleic acid coding for a protein.

12. Process for introducing nucleic acid(s) into human or animal cells in vitro by transferrin receptor-mediated endocytosis, wherein a complex which is preferably soluble under physiological conditions is formed from transferrin-polycation conjugate and nucleic acid or acids and the cells are brought into contact with this complex, characterised in that a conjugate as defined in claim 1 is used.

13. Pharmaceutical preparation containing one or more of the complexes defined in claims 4 to 11.

## Revendications

1. Conjugué consistant en transferrine et en un polycation couplé à celle-ci de manière covalente caractérisé en ce que le polycation est un polypeptide homologue synthétique ou une polyéthylèneimine.

2. Conjugué selon la revendication 1, caractérisé en ce que le polypeptide homologue synthétique est une polylysine.

3. Conjugué selon la revendication 1 ou 2, caractérisé en ce que le polycation présente environ 20 à environ 500 charges positives.

4. Complexe transferrine-polycation/acide nucléique qui est absorbé dans la cellule humaine ou animale par endocytose à médiation par le récepteur de la transferrine, contenant un conjugué selon l'une des revendications 1 à 3.

5. Complexe selon la revendication 4 caractérisé en ce qu'il contient comme acide nucléique un acide nucléique capable d'inhiber spécifiquement des gènes ou la fonction d'ARN.

6. Complexe selon la revendication 5 caractérisé en ce qu'il contient un acide nucléique qui est capable d'inhiber un acide nucléique viral.

7. Complexe selon la revendication 5 caractérisé en ce qu'il contient un acide nucléique qui est capable d'inhiber des oncogènes ou d'autres gènes clés, qui contrôlent la croissance et/ou la différenciation de cellules.

8. Complexe selon l'une des revendications 5 à 7 caractérisé en ce qu'il contient comme acide nucléique un ribozyme, le cas échéant avec un ARN porteur, ou le gène le codant.

9. Complexe selon la revendication 8 caractérisé en ce qu'il contient comme acide nucléique une unité génétique consistant en un gène d'ARNt comme gène porteur et en un gène de ribozyme disposé dans ce gène.

10. Complexe selon l'une des revendications 5 à 7 caractérisé en ce qu'il contient comme acide nucléique un oligonucléotide antisens, le cas échéant avec un acide nucléique porteur, ou dans le cas d'un oligonucléotide d'ARN le gène le codant.

11. Complexe selon la revendication 4 caractérisé en ce qu'il contient un acide nucléique codant une protéine.

12. Procédé d'introduction d'acide(s) nucléique(s) dans des cellules humaines ou animales in vitro par endocytose à médiation par le récepteur de la transferrine, où on forme à partir d'un conjugué transferrine-polycation et d'acide(s) nucléique(s) un complexe, de préférence soluble dans les conditions physiologiques, et on met les cellules en contact avec ce complexe, caractérisé en ce que l'on utilise un conjugué défini dans la revendication 1.

13. Préparation pharmaceutique contenant un ou plusieurs des complexes définis dans les revendications 4 à 11.
